(19) Europäisches Patentamt
European Patent Office
Office européen des brevets

(11) Numéro de publication : **0 397 556 B1**

(12) # FASCICULE DE BREVET EUROPEEN

(45) Date de publication du fascicule du brevet : **20.10.93 Bulletin 93/42**

(21) Numéro de dépôt : **90401218.4**

(22) Date de dépôt : **09.05.90**

(51) Int. Cl.5 : **C07C 275/28,** C07C 275/34, C07C 237/24, C07C 323/63, C07D 209/42, A61K 31/405, A61K 31/395, A61K 31/16

(54) **N-Phényl amides, leurs procédés de préparation et les médicaments les contenant.**

Le dossier contient des informations techniques présentées postérieurement au dépôt de la demande et ne figurant pas dans le présent fascicule.

(30) Priorité : **12.05.89 FR 8906250**

(43) Date de publication de la demande : **14.11.90 Bulletin 90/46**

(45) Mention de la délivrance du brevet : **20.10.93 Bulletin 93/42**

(84) Etats contractants désignés : **AT BE CH DE DK ES FR GB GR IT LI LU NL SE**

(56) Documents cités :
**EP-A- 0 250 148**
**EP-A- 0 272 228**
**EP-A- 0 308 885**

(73) Titulaire : **RHONE-POULENC SANTE**
**20, avenue Raymond Aron**
**F-92160 Antony (FR)**

(72) Inventeur : **Bourzat, Jean-Dominique**
**20 Boulevard de la Libération**
**F-94300 Vincennes (FR)**
Inventeur : **Capet, Marc**
**10 rue de la Galaise**
**F-94320 Thiais (FR)**
Inventeur : **Cotrel, Claude**
**17A Avenue du Docteur Arnold Netter**
**F-75012 Paris (FR)**
Inventeur : **Guyon, Claude**
**17 Bis Avenue Henri Martin**
**F-94100 Saint Maur des Fosses (FR)**
Inventeur : **Manfre, Franco**
**40 rue Clément Perrot**
**F-94400 Vitry Sur Seine (FR)**
Inventeur : **Roussel, Gérard**
**20 Ter Rue des Carrières**
**F-91450 Soisy sur Seine (FR)**

(74) Mandataire : **Savina, Jacques et al**
**RHONE-POULENC SANTE, Service Brevets,**
**20 Avenue Raymond Aron**
**F-92165 Antony Cédex (FR)**

EP 0 397 556 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

EP - A - 0 250 148 décrit des analogues de l'acide glutamique et d'autres acides aminés qui sont utiles dans la prévention et le traitement des désordres liés à la cholécystokinine au niveau du système nerveux, de l'appareil gastro-intestinal et comme régulateurs de l'appetit.

La présente invention concerne des dérivés de formule :

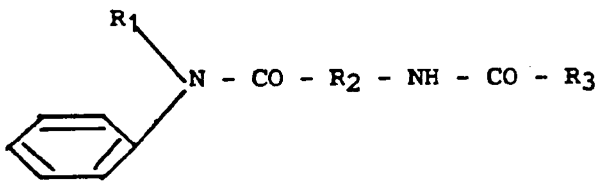

(I)

leurs procédés de préparation et les médicaments les contenant.

Dans la formule (I),

- $R_1$ représente :
  - un radical phényle ou phényle substitué par un radical alkyle, alcoxy, cyano ou par un atome d'halogène,
  - une chaîne $-CH(R_8)-COOR_4$ dans laquelle $R_4$ représente un atome d'hydrogène ou un radical alkyle, cycloalkyle, cycloalkylalkyle, phénylalkyle ou phényle et $R_8$ représente un atome d'hydrogène ou un radical alkyle ou phényle,
  - une chaîne $-CH_2-CO-NR_5R_6$ dans laquelle $R_5$ et $R_6$ identiques ou différents représentent un radical alkyle ou forment avec l'atome d'azote auquel ils sont rattachés un radical pyrrolidinyl-1 éventuellement substitué par un radical alkyle ou
  - un radical phénylalkyle,
- $R_2$ représente un radical méthylène, éthylène ou $-CH(R_7)-$ dans lequel $R_7$ représente un radical alkyle, benzyle, alkylthioalkyle dont les portions alkyle contiennent 1 ou 2 atomes de carbone ou phényle,
- $R_3$ représente un radical naphtyl-1 ou -2, indolyl-2 ou -3 ou phénylamino dont le noyau phényle est éventuellement substitué par un radical alkyle, alcoxy, nitro, hydroxy ou alkylthio ou par 1 ou 2 atomes d'halogène,

étant entendu que lorsque $R_3$ représente un radical naphtyl-1 ou -2 ou indolyl-2 ou -3, $R_7$ ne peut pas représenter un radical benzyle, alkylthioalkyle ou phényle.

Dans les définitions qui précèdent et celles qui seront citées ci-après, sauf mention contraire, les radicaux alkyle et alcoxy et les portions alkyle et alcoxy contiennent 1 à 4 atomes de carbone en chaîne droite ou ramifiée.

Dans la formule (I), les atomes d'halogène sont de préférence des atomes de chlore, de brome ou de fluor.

Les composés de formule (I) pour lesquels $R_2$ représente un radical $-CH(R_7)-$ présentent des formes isomères. Les racémiques et les énantiomères de ces composés font également partie de l'invention.

Les composés de formule (I) pour lesquels

- $R_2$ représente un radical méthylène, éthylène ou $-CH(R_7)-$ dans lequel $R_7$ représente un radical alkyle,
- $R_3$ représente un radical phénylamino dont le noyau phényle est éventuellement substitué par un radical alkyle, alcoxy, nitro, alkylthio ou par 1 ou 2 atomes d'halogène et
- $R_1$ a les mêmes significations que précédemment,

peuvent être préparés par action d'un dérivé de formule :

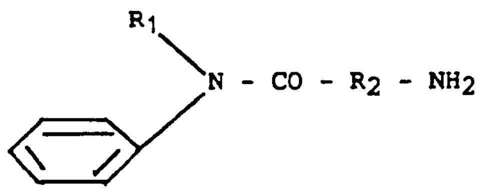

(II)

dans laquelle $R_2$ a les mêmes significations que précédemment et $R_1$ a les mêmes significations que dans la formule (I) sur un phénylisocyanate dont le noyau phényle est éventuellement substitué par un radical alkyle, alcoxy, nitro, alkylthio ou par 1 ou 2 atomes d'halogène.

Cette réaction s'effectue généralement au sein d'un solvant organique inerte tel que le tétrahydrofuranne, le diméthylformamide, un solvant chloré (chloroforme, chlorure de méthylène par exemple), un solvant aromatique (benzène, toluène par exemple), à une température comprise entre 10°C et la température d'ébullition du solvant.

Les phénylisocyanates dont le noyau phényle est substitué par un radical alkyle, alcoxy, nitro, alkylthio ou par 1 ou 2 atomes d'halogène peuvent être obtenus par application ou adaptation de la méthode décrite par R. RICHTER et al. The Chemistry of cyanate and their thio derivatives, S. Patai, part 2, Wiley New York (1977).

Les dérivés de formule (II) peuvent être obtenus par application ou adaptation de la méthode décrite par T. WIELAND et al., Justus Liebigs Ann. Chem., 613, 84 (1958) ou par adaptation de la méthode de GABRIEL (GIBSON et al., Angew. Chem. Int. Ed., 7,919 (1968) qui consiste à faire réagir l'hydrate d'hydrazine sur un composé de formule :

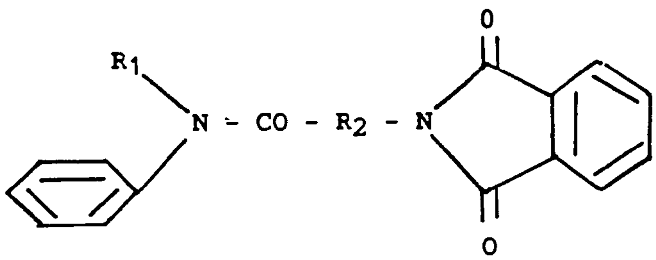

(III)

dans laquelle $R_1$ et $R_2$ ont les mêmes significations que dans la formule (II).

Cette réaction s'effectue de préférence au sein d'un solvant inerte tel qu'un alcool (méthanol, éthanol, propanol par exemple), à une température comprise entre 0°C et la température d'ébullition du solvant.

Les dérivés de formule (III), à l'exception de ceux pour lesquels $R_1$ représente soit une chaîne $-CH(R_8)-COOR_4$ dans laquelle $R_4$ représente un atome d'hydrogène soit une chaîne $-CH_2-CO-HR_5R_6$, peuvent être obtenus par action du phtalimide potassé sur un dérivé de formule :

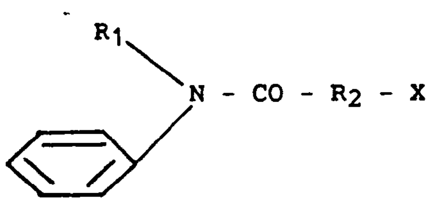

(IV)

dans laquelle $R_1$ a les mêmes significations que ci-dessus, $R_2$ a les mêmes significations que dans la formule (III) et X représente un atome d'halogène, de préférence chlore ou brome.

Cette réaction s'effectue généralement au sein d'un solvant inerte tel que le diméthylformamide, un solvant aromatique (toluène, xylène par exemple), à une température voisine de 100°C.

Les dérivés de formule (IV) peuvent être obtenus par action d'une amine de formule :

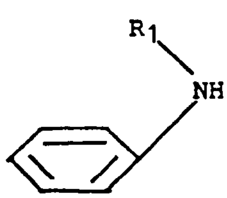

(V)

dans laquelle $R_1$ a les mêmes significations que dans la formule (IV) sur un dérivé dihalogéné de formule :

$$X\text{-}CO\text{-}R_2\text{-}X \quad (VI)$$

dans laquelle $R_2$ a les mêmes significations que dans la formule (IV) et X représente un atome d'halogène, de préférence chlore ou brome.

Cette réaction s'effectue de préférence en présence d'une amine tertiaire telle que la triéthylamine, au sein d'un solvant chloré (chloroforme, chlorure de méthylène, dichloro-1,2 éthane par exemple), à une température comprise entre 0°C et la température d'ébullition du solvant.

Les amines de formule (V), pour lesquelles $R_1$ représente un radical phényle substitué par un radical alkyle, alcoxy, cyano ou par un atome d'halogène, peuvent être préparées par application ou adaptation des méthodes décrites par K. NAKAMURA et al., Synthesis, 882 (1974) ; J. KULAGOWSKI et al., J. Chem. Soc. Perkin Trans I, 2725 (1985), I. GOLDBERG, Chem. Ber., S.40, 4541 (1907) et R. WILLSTATTER et al., Chem. Ber., 42, 4135 (1909).

Les amines de formule (V), pour lesquelles $R_1$ représente un radical phénylalkyle peuvent être obtenues par application ou adaptation des méthodes décrites par SPRINZAK, J. Am. Chem. Soc., 78, 3207 (1956) ou SCHELLENBERG, J. Org. Chem., 28, 3259 (1963).

Les amines de formule (V), pour lesquelles $R_1$ représente une chaîne $-CH(R_8)-COOR_4$ dans laquelle $R_4$ représente un radical alkyle, cycloalkyle, cycloalkylalkyle, phénylalkyle ou phényle et $R_8$ représente un atome d'hydrogène, peuvent être obtenues par action de l'aniline sur un dérivé halogéné de formule :

$$X - CH_2 - COOR_4 \qquad (VII)$$

dans laquelle $R_4$ a les mêmes significations que précédemment et X représente un atome d'halogène, de préférence chlore ou brome.

Cette réaction s'effectue généralement dans un solvant inerte tel qu'un solvant chloré (chloroforme, dichloro-1,2 éthane, chlorure de méthylène par exemple), à la température d'ébullition du solvant.

Les amines de formule (V), pour lesquelles $R_1$ représente une chaîne $-CH(R_8)-COOR_4$ dans laquelle $R_8$ représente un radical alkyle ou phényle, peuvent être obtenues par application ou adaptation de la méthode décrite par M. JULIA et al., Bull. Soc. Chim. France, 661 (1958).

Les dérivés halogénés de formule (VII) peuvent être obtenus par application ou adaptation de la méthode décrite par H.J. BACKER et al., Recueil Trav. Chim. Pays-Bas, 58, 1048 (1939) et dans les exemples.

Les dérivés dihalogénés de formule (VI), pour lesquels $R_2$ représente un radical $-CH(R_7)$ dans lequel $R_7$ représente un radical alkyle, peuvent être préparés par halogénation et de préférence par bromuration ou chloruration de l'acide correspondant.

L'halogénation s'effectue par toute méthode connue de l'homme du métier pour passer d'un acide à un halogènure d'acide. Il est particulièrement avantageux d'opérer au moyen de tribromure de phosphore, d'oxybromure de phosphore ou de chlorure de thionyle dans un solvant inerte tel que le benzène, le toluène, le chloroforme, à une température comprise entre 10°C et la température d'ébullition du solvant.

Les dérivés de formule (III), pour lesquels $R_1$ représente une chaîne $-CH(R_8)-COOR_4$ dans laquelle $R_4$ représente un atome d'hydrogène, peuvent être obtenus par hydrolyse du dérivé de formule (III) correspondant pour lequel $R_1$ représente une chaîne $-CH(R_8)-COOR_4$ dans laquelle $R_4$ représente un radical alkyle.

Cette hydrolyse s'effectue par toute méthode connue de l'homme du métier pour passer d'un ester à un acide carboxylique sans toucher au reste de la molécule. Il est particulièrement avantageux d'opérer au moyen d'acide trifluoroacétique, au sein d'un solvant inerte chloré tel que le chloroforme, le dichlorométhane ou le dichloro-1,2 éthane, à la température d'ébullition du solvant.

Les dérivés de formule (III) pour lesquels $R_1$ représente un chaîne $-CH_2-CO-NR_8R_6$ peuvent être obtenus par action d'une amine de formule :

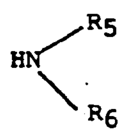

(VIII)

dans laquelle $R_8$ et $R_6$ identiques ou différents représentent un radical alkyle ou forment avec l'atome d'azote auquel ils sont rattachés un radical pyrrolidinyl-1 éventuellement substitué par un radical alkyle , sur un dérivé de formule (III) correspondant pour lequel $R_1$ représente une chaîne $-CH(R_8)-COOR_4$ dans laquelle $R_4$ et $R_8$ représentent un atome d'hydrogène ou un dérivé réactif de cet acide.

Lorsque l'on met en oeuvre l'acide on opère en présence d'un agent de condensation peptidique tel qu'un carbodiimide (par exemple le dicyclohexylcarbodiimide) ou le N,N'-diimidazole carbonyle, dans un solvant inerte tel qu'un éther (par exemple THF, dioxanne) un amide (par exemple DMF) ou un solvant chloré (par exemple chlorure de méthylène, dichloroéthane, chloroforme) à une température comprise entre 0°C et la température de reflux du mélange réactionnel.

Lorsque l'on met en oeuvre un dérivé réactif de l'acide il est possible de faire réagir l'anhydride, un anhydride mixte, un halogénure d'acide ou un ester (qui peut être choisi parmi les esters activés ou non de l'acide).

On opère alors soit en milieu organique, éventuellement en présence d'un accepteur d'acide tel qu'une base organique azotée (par exemple une trialkylamine, une pyridine, le diaza-1,8 bicyclo-[5.4.0] undécène-7 ou le diaza-1,5 bicyclo [4.3.0] nonène-5), dans un solvant tel que cité ci-dessus, ou un mélange de ces solvants,

à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les dérivés de formule (III) pour lesquels $R_1$ représente un radical phényle éventuellement substitué peuvent également être obtenus par action d'une amine de formule (V) sur un dérivé de formule :

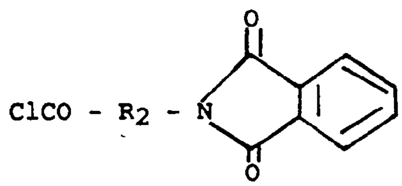

(IX)

dans laquelle $R_2$ a les mêmes significations que dans la formule (I).

Cette réaction s'effectue de préférence au sein d'un solvant inerte tel qu'un solvant chloré (chlorure de méthylène, dichloroéthane, chloroforme par exemple), à une température comprise entre 15 et 70°C.

Les dérivés de formule (IX) peuvent être préparés par application ou adaptation de la méthode décrite par W. GRASSMANN et al., Chem. Ber., 83, 244 (1950).

Les composés de formule (I), pour lesquels

- $R_2$ représente un radical méthylène, éthylène ou $-CH(R_7)-$ dans lequel $R_7$ représente un radical alkyle,
- $R_3$ représente un radical phénylamino dont le noyau phényle est éventuellement substitué par un radical alkyle, alcoxy, alkylthio, nitro, hydroxy ou par 1 ou 2 atomes d'halogène et
- $R_1$ a les mêmes significations que précédemment,

peuvent être préparés par action d'un dérivé de formule :

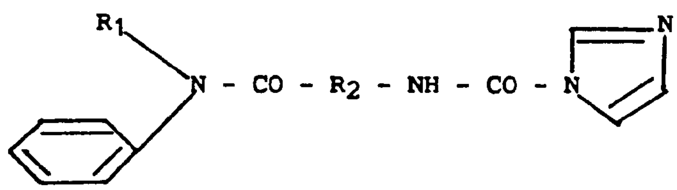

(X)

dans laquelle $R_1$ et $R_2$ ont les mêmes significations que précédemment, sur une aniline éventuellement substituée par un radical alkyle, alcoxy, alkylthio, nitro, hydroxy ou par 1 ou 2 atomes d'halogène.

Cette réaction s'effectue généralement dans un solvant inerte tel que le tétrahydrofuranne, le diméthylformamide, un solvant chloré (chloroforme, dichlorométhane, dichloro-1,2 éthane par exemple), un solvant aromatique (benzène, toluène par exemple), à une température comprise entre 20°C et la température d'ébullition du solvant.

Les anilines substituées peuvent être obtenues par application ou adaptation de la méthode décrite par R. SCHRÖTER, Methoden der Organischen Chemie, Houben Weil, Band XI/1 p. 360-380.

Les dérivés de formule (X) peuvent être obtenus par action d'un dérivé de formule (II) sur le N,N'-diimidazolecarbonyle.

Cette réaction s'effectue dans les mêmes solvants que ceux mentionnés précédemment pour la réaction d'un composé de formule (X) sur une aniline, à une température comprise entre 15 et 50°C.

Il est possible de ne pas isoler le composé de formule (X) et de le faire réagir in situ avec une aniline.

Les composés de formule (I), pour lesquels

- $R_2$ représente un radical méthylène, éthylène ou $-CH(R_7)-$ dans lequel $R_7$ représente un radical alkyle,
- $R_3$ représente un radical naphtyl-1 ou -2 ou indolyl-2 ou -3 et
- $R_1$ a les mêmes significations que précédemment,

peuvent être obtenus par action d'un dérivé de formule (II) dans laquelle $R_1$ et $R_2$ ont les mêmes significations que ci-dessus, sur un dérivé de formule :

$$H00C - R_3 \qquad (XI)$$

dans laquelle $R_3$ représente un radical naphtyl-1 ou -2 ou indolyl-2 ou -3 ou un dérivé réactif de cet acide.

Cette réaction s'effectue dans les mêmes conditions que celles décrites précédemment pour la réaction d'une amine de formule (VIII) sur un dérivé de formule (III).

Les composés de formule (I), pour lesquels

- $R_3$ représente un radical phénylamino dont le noyau phényle est éventuellement substitué par un radical alkyle, alcoxy, nitro, alkylthio ou par 1 ou 2 atomes d'halogène,
- $R_2$ a les mêmes significations que précédemment,
- $R_1$ a les mêmes significations que précédemment à l'exception de représenter une chaîne - $CH(R_8)-COOR_4$ dans laquelle $R_4$ représente un atome d'hydrogène ou une chaîne - $CH_2-CO-NR_5R_5$,

peuvent être obtenus par action d'une amine de formule (V) dans laquelle $R_1$ a les mêmes significations que dans la formule (I) sur un dérivé de formule :

$$HOOC - R_2 - NH - CO - R_3 \qquad (XII)$$

dans laquelle $R_3$ représente un radical phénylamino dont le noyau phényle est éventuellement substitué par un radical alkyle, alcoxy, nitro, alkylthio ou par 1 ou 2 atomes d'halogène et $R_2$ a les mêmes significations que dans la formule (I).

Cette réaction s'effectue de préférence en présence de chlorure de thionyle, au sein d'un solvant chloré (chloroforme, chlorure de méthylène, dichloro-1,2 éthane par exemple), à la température d'ébullition du solvant.

Les dérivés de formule (XII) peuvent être obtenus par action d'un aminoacide de formule :

$$HOOC - R_2 - NH_2 \qquad (XIII)$$

dans laquelle $R_2$ a les mêmes significations que dans la formule (XII) sur un isocyanate de phényle dont le noyau phényle est éventuellement substitué par un radical alkyle, alcoxy, nitro, alkylthio ou par 1 ou 2 atomes d'halogène.

Cette réaction s'effectue généralement dans l'eau, en présence d'une base telle qu'un carbonate de métal alcalin (bicarbonate de sodium ou de potassium par exemple), à une température voisine de 20°C.

Les composés de formule (I), pour lesquels,

- $R_3$ représente un radical phénylamino dont le noyau phényle est éventuellement substitué par un radical alkyle, alcoxy, nitro, alkylthio ou par 1 ou 2 atomes d'halogène,
- $R_2$ a les mêmes significations que précédemment et,
- $R_1$ représente une chaîne - $CH(R_5)-COOR_4$ dans laquelle $R_4$ représente un atome d'hydrogène

peuvent être obtenus par hydrolyse du dérivé de formule (I) correspondant pour lequel $R_1$ représente une chaîne - $CH(R_8)-COOR_4$ dans laquelle $R_4$ représente un radical alkyle.

Cette hydrolyse s'effectue par toute méthode connue de l'homme du métier pour passer d'un ester à un acide carboxylique sans toucher au reste de la molécule. Il est particulièrement avantageux d'opérer au moyen d'acide trifluoroacétique, au sein d'un solvant inerte chloré tel que le chloroforme, le dichlorométhane ou le dichloro-1,2 éthane, à la température d'ébullition du solvant.

Les composés de formule (I), pour lesquels,

- $R_3$ représente un radical phénylamino dont le noyau phényle est éventuellement substitué par un radical alkyle, alcoxy, nitro, alkylthio ou par 1 ou 2 atomes d'halogène,
- $R_2$ a les mêmes significations que précédemment et,
- $R_1$ représente une chaîne - $CH_2-CO-NR_5R_5$

peuvent être obtenus par action d'une amine de formule (VIII) sur un acide de formule (I) correspondant pour lequel $R_1$ représente une chaîne - $CH(R_5)-COOR_4$ dans laquelle $R_4$ représente un atome d'hydrogène ou un dérivé réactif de cet acide.

Cette réaction s'effectue dans les conditions décrites précédemment pour la préparation des composés de formule (III) dans laquelle $R_1$ représente une chaîne - $CH_2-CO-NR_5R_5$.

Les composés de formule (I), pour lesquels $R_3$ représente un radical phénylamino dont le noyau phényle est substitué par un radical hydroxy, peuvent être obtenus par désalkylation d'un dérivé de formule (I) correspondant pour lequel $R_3$ représente un radical phénylamino dont le noyau phényle est substitué par un radical alcoxy.

Cette désalkylation s'effectue généralement au moyen d'une solution chlorométhylénique de tribromure de bore au sein d'un solvant chloré (chloroforme, chlorure de méthylène, dichloro-1,2 éthane par exemple), à une température comprise entre -50°C et +20°C.

Les composés de formule (I) pour lesquels $R_1$ représente une chaîne - $CH(R_5)$ - $COOR_4$ dans laquelle $R_4$ représente un radical phényle peuvent également être préparés par action de phénol sur un composé de formule (I) correspondant pour lequel $R_4$ représente un atome d'hydrogène.

Cette réaction s'effectue généralement au sein d'un solvant inerte tel qu'un solvant chloré (chloroforme, chlorure de méthylène par exemple) en présence d'une base telle qu'une trialkylamine, et d'hexafluorophosphonate de benzotriazolyl-1 oxy-tris(diméthylamino) phosphonium, à une température voisine de 25°C.

Les énantiomères des composés de formule (I) pour lesquels $R_2$ représente un radical $-CH(R_7)-$ peuvent être obtenus par dédoublement des racémiques par exemple par chromatographie sur colonne chirale selon W.H. PIRCKLE et al., asymetric synthesis, vol.1, Academic Press (1983) ou par synthèse à partir des précurseurs chiraux.

Les composés de formule (I) peuvent être purifiés par les méthodes connues habituelles, par exemple par cristallisation, chromatographie ou extractions.

Les composés de formule (I) présentent des propriétés pharmacologiques intéressantes. Ces composés possédent une forte affinité pour les récepteurs de cholécystokinine (CCK) et sont donc utiles dans le traitement et la prévention des désordres liés à la CCK au niveau du système nerveux et de l'appareil gastrointestinal.

C'est ainsi que ces composés peuvent être utilisés pour le traitement ou la prévention des psychoses, des troubles anxieux de la maladie de Parkinson, de la diskinésie tardive, du syndrôme du colon irritable, de la pancréatite aigüe, des ulcères, des désordres de la motilité intestinale, de certaines tumeurs de l'oesophage inférieur, du colon et de l'intestin et comme régulateur de l'appétit.

Ces composés ont également un effet de potentialisation sur l'activité analgésique des médicaments narcotiques et non narcotiques.

L'affinité des composés de formule (I) pour les récepteurs CCK a été déterminée selon une technique inspirée de celle de A. SAITO et al., J. Neuro. Chem., 37, 483-490 (1981).

Dans ce test, la $CI_{50}$ des composés de formule (I) est inférieure ou égale à 1000 nM.

Les composés de formule (I) présentent une toxicité faible. Leur $DL_{50}$ est généralement supérieure à 40 mg/kg par voie sous cutanée chez la souris.

D'un intérêt particulier sont les composés de formule (I) pour lesquels $R_1$ représente un radical phényle éventuellement substitué par un radical alcoxy ou un atome d'halogène, une chaîne -$CH(R_8)$-$COOR_4$ dans laquelle $R_4$ représente un radical alkyle ou cycloalkylalkyle et $R_8$ représente un atome d'hydrogène ou un radical phényle et $R_2$ et $R_3$ sont tels que définis précédemment.

Les composés préférés sont les suivants :

- [(méthylthio-3 phényl)-3 uréido]-2 N,N-diphényl acétamide,
- [(méthyl-3 phényl)-3 uréido]-3 N,N-diphényl propionamide,
- {[(méthyl-3 phényl)-3 uréido]-2 N-phénylacétamido}-2 acétate de tert-butyle,
- N-{[(méthyl-3 phényl)-3 uréido]-2 phényl-3 propionyl} N-phényl glycinate de tert-butyle-(RS),
- [(dichloro-2,3 phényl)-3 uréido]-2 N,N-diphényl acétamide,
- {[méthyl-3 phényl)-3 uréido]-2 N-phényl acétamido}-2 phénylacétate d'éthyle-(RS),
- N-(chloro-3 phényl)[(méthyl-3 phényl)-3 uréido]-2 N-phényl acétamide,
- N-(methoxy-4 phényl) N-phényl [(méthyl-3 phényl)-3 uréido]-2 acétamide,
- N-{[(méthylthio-3 phényl)-3 uréido]-2 acétyl}N-phényl glycinate de tert-butyle,
- N-{[(méthoxy-3 phényl)-3 uréido]-2 acétyl} N-phényl glycinate de tert-butyle,
- N-(N-tert-butoxycarbonylméthyl phénylcarbamoylméthyl) indolecarboxamide-2,
- N-[N-(dichloro-3,4 benzoyl) glycyl] N-phényl glycinate de tert-butyle,
- N-[N-(naphtoyl-2) glycyl] N-phényl glycinate de tert-butyle,
- {[(méthyl-3 phényl)-3 uréido]-2 N-phényl acétamido}-2 acétate de cyclopropylméthyle,
- {[(méthyl-3 phényl]-3 uréido]-2 N-phényl acétamido}-2 acétate de sec-butyle-(RS).

Les exemples suivants illustrent l'invention sans la limiter.

## EXEMPLE 1

A une solution de 1 g d'amino-2 N,N-diphényl acétamide dans 20 cm3 de tétrahydrofuranne anhydre, on ajoute, à une température voisine de 25°C, 0,59 g d'isocyanate de méthyl-3 phényle. La suspension obtenue est agitée 4 heures à une température voisine de 25°C et le produit insoluble est séparé par filtration. On obtient, après recristallisation dans l'acétonitrile, 0,8 g de N,N-diphényl [(méthyl-3 phényl)-3 uréido]-2 acétamide fondant à 240°C.

L'amino-2 N,N-diphényl acétamide peut être préparé selon la méthode décrite par T. WEILAND et H. URBACH, Justus Liebigs Ann. Chem., 613, 84 (1958).

## EXEMPLE 2

En opérant d'une manière analogue à celle décrite à l'exemple 1, mais à partir de 1 g d'amino-2 N,N-diphényl acétamide et de 0,59 g d'isocyanate de phényle, on obtient, après recristallisation dans un mélange de diméthylformamide et d'acétonitrile (20-80 en volumes), 1,2 g de N,N-diphényl (phényl-3 uréido)-2 acétamide fondant à 240°C.

EXEMPLE 3

En opérant d'une manière analogue à celle décrite à l'exemple 1, mais à partir de 2 g d'amino-2 N,N-diphényl acétamide et de 1,3 g d'isocyanate de méthoxy-3 phényle, on obtient, après recristallisation dans l'acétonitrile, 2,6 g de [(méthoxy-3 phényl)-3 uréido]-2 N,N-diphényl acétamide fondant à 182°C.

EXEMPLE 4

A une solution de 1,6 g de [(méthoxy-3 phényl)-3 uréido]-2 N,N-diphényl acétamide dans 25 cm3 de dichlorométhane maintenue sous atmosphère d'azote, on ajoute en 15 minutes, à une température voisine de -50°C, 25 cm3 d'une solution chlorométhylénique 1 M de tribromure de bore. Le mélange obtenu est agité 2 heures à une température voisine de -50°C, puis 20 heures à une température voisine de 20°C. Le précipité formé est séparé par filtration, lavé par 6 fois 10 cm3 d'eau distillée et séché à l'air. On obtient, après recristallisation dans l'acétonitrile, 0,5 g d'[(hydroxy-3 phényl)-3 uréido]-2 N,N-diphényl acétamide fondant à 244°C.

EXEMPLE 5

A une solution de 1 g d'amino-2 N,N-diphényl acétamide dans 15 cm3 de tétrahydrofuranne anhydre, on ajoute 0,72 g de N,N'-diimidazole carbonyle. La solution est agitée 2 heures à une température voisine de 25°C puis on ajoute 1,2 g de méthylthio-3 aniline. La solution obtenue est agitée à reflux pendant 12 heures puis concentrée à sec sous pression réduite (2,7 kPa). Le résidu obtenu est repris avec 60 cm3 d'eau distillée et agité pendant 30 minutes à une température voisine de 20°C. Le produit insoluble est séparé par filtration, lavé à l'eau distillée et séché à l'air. On obtient, après recristallisation dans l'acétonitrile, 1 g de [(méthylthio-3 phényl)-3 uréido]-2 N,N-diphényl acétamide fondant à 182°C.

EXEMPLE 6

A une solution de 3,2 g de N-(diphénylcarbamoylméthyl) imidazolecarboxamide-1 dans 35 cm3 de toluène anhydre, on ajoute 2,8 g de nitro-3 aniline. La solution obtenue est agitée à reflux pendant 4 heures. Après refroidissement, le mélange réactionnel est lavé avec 40 cm3 d'une solution aqueuse d'acide méthanesulfonique N et 2 fois 30 cm3 d'eau distillée. La phase organique est séchée sur sulfate de magnésium, filtrée et concentrée à sec sous pression réduite (2,7 kpa) à 40°C. Le résidu est agité pendant 20 minutes dans 25 cm3 de dichlorométhane. Le produit insoluble est séparé par filtration, lavé avec 5 cm3 de dichlorométhane et séché à l'air. On obtient, après recristallisation dans l'acétate d'éthyle 0,7 g de [(nitro-3 phényl)-3 uréido]-2 N,N-diphényl acétamide fondant à 180°C.

Le N-(diphénylcarbamoylméthyl) imidazolecarboxamide-1 peut être préparé de la manière suivante : à une solution de 7,3 g de N,N′-diimidazole carbonyle dans 100 cm3 de tétrahydrofuranne anhydre, on coule une solution de 10,2 g d'amino-2 N,N-diphényl acétamide dans 30 cm3 de tétrahydrofuranne anhydre. La solution obtenue est agitée pendant 3 heures à une température voisine de 25°C, puis concentrée à sec sous pression réduite (2,7 kPa) à 40°C. Le résidu est dissous dans 150 cm3 d'acétate d'éthyle et la solution obtenue est lavée par 2 fois 100 cm3 d'eau distillée, séchée sur sulfate de magnésium et concentrée à sec sous pression réduite (2,7 kPa) à 40°C. On obtient ainsi 14 g de N-(diphénylcarbamoylméthyl) imidazolecarboxamide-1 fondant à 162°C.

EXEMPLE 7

En opérant d'une manière analogue à celle décrite à l'exemple 1, mais à partir de 1,2 g d'amino-2 N,N-diphényl propionamide (RS) et de 0,66 g d'isocyanate de méthyl-3 phényle, on obtient, après recristallisation dans le méthanol, 0,8 g de [(méthyl-3 phényl)-3 uréido]-2 N,N-diphényl propionamide - (RS) fondant à 197°C.

L'amino-2 N,N-diphényl propionamide - (RS) peut être préparé de la manière suivante : à une solution de 10,7 g de phtalimido-2 N,N-diphényl propionamide - (RS) dans 150 cm3 de méthanol, on ajoute 2,9 g d'hydrate d'hydrazine. La solution obtenue est agitée à reflux pendant 3 heures, puis versée dans 150 cm3 d'une solution aqueuse d'acide chlorhydrique 4N. Le produit insoluble est séparé par filtration et le filtrat est concentré jusqu'à un volume d'environ 120 cm3, sous pression réduite (2,7 kPa) à 40°C. Après neutralisation avec une solution aqueuse de soude 4N, la solution est extraite par 3 fois 50 cm3 d'acétate d'éthyle. Les phases organiques réunies sont lavées par 4 fois 50 cm3 d'eau distillée, séchées sur sulfate de magnésium puis concentrée à sec sous pression réduite (2,7 kPa) a 40°C. On obtient ainsi 5,8 g d'amino-2 N,N-diphényl propionamide - (RS) fondant à 82°C.

Le phtalimido-2 N,N-diphényl propionamide - (RS) peut être préparé de la manière suivante : à une solution de 8,9 g de chloro-2 N,N-diphényl propionamide - (RS) dans 160 cm3 de diméthylformamide, on ajoute 12,6 g de phtalimide potassé. Le mélange est agité à une température voisine de 100°C pendant 5 heures, puis versé dans 1500 cm3 d'eau distillée. Le produit insoluble est séparé par filtration, lavé par 3 fois 60 cm3 d'eau distillée et séché à l'air. On obtient ainsi 10,7 g de phtalimido-2 N,N-diphényl propionamide (RS) fondant à 152°C.

Le chloro-2 N,N-diphényl propionamide - (RS) peut être préparé de la manière suivante : à une solution de 10,2 g de diphénylamine et de 8 g de triéthylamine dans 60 cm3 de dichloro-1,2 éthane maintenue à une température voisine de 15°C, on ajoute 10,2 g de chlorure de chloro-2 propionyle. Le mélange est agité pendant 6 heures à une température voisine de 60°C. Après refroidissement, il est lavé avec 3 fois 100 cm3 d'eau distillée. La phase organique est séchée sur sulfate de magnésium et concentrée à sec sous pression réduite (2,7 kPa) à 40°C. On obtient ainsi 8,9 g de chloro-2 N,N-diphényl propionamide - (RS) fondant à 89°C.

EXEMPLE 8

En opérant d'une manière analogue à celle décrite à l'exemple 1, mais à partir de 1 g d'amino-3 N,N-diphényl propionamide et de 0,55 g d'isocyanate de méthyl-3 phényle, on obtient, après recristallisation dans l'acétonitrile, 0,85 g de [(méthyl-3 phényl)-3 uréido]-3 N,N-diphényl propionamide fondant à 179°C.

L'amino-3 N,N-diphényl propionamide peut être préparé d'une manière analogue à celle décrite à l'exemple 7 pour la préparation de l'amino-2 N,N-diphényl propionamide, mais à partir de 8,4 g de phtalimido-3 N,N-diphényl propionamide et de 2,3 g d'hydrate d'hydrazine. On obtient ainsi 3,9 g d'amino-3 N,N-diphényl propionamide fondant à 50°C.

Le phtalimido-3 N,N-diphényl propionamide peut être préparé d'une manière analogue à celle décrite à l'exemple 7 pour la préparation du phtalimido-2 N,N-diphényl propionamide (RS), mais à partir de 6,3 g de chloro-3 N,N-diphényl propionamide et de 9 g de phtalimide potassé. On obtient ainsi 8,5 g de phtalimido-3 N,N-diphényl propionamide fondant à 140°C.

Le chloro-3 N,N-diphényl propionamide peut être préparé d'une manière analogue à celle décrite à l'exemple 7 pour la préparation du chloro-2 N,N-diphényl propionamide - (RS), mais à partir de 8,5 g de diphénylamine, de 7,1 g de triéthylamine et de 8,8 g de chlorure de chloro-3 propionyle. On obtient ainsi 6,3 g de chloro-3 N,N-diphényl propionamide fondant à 92°C.

EXEMPLE 9

A une solution de 2,15 g d'acide indolecarboxylique-2 et de 0,1 cm3 de diméthylformamide dans 30 cm3 d'éther diéthylique anhydre maintenue sous atmosphère d'azote, on ajoute, à une température voisine de 10°C, 1,7 g de dichlorure d'oxalyle dissous dans 5 cm3 d'éther diéthylique anhydre. La solution obtenue est agitée 2 heures à une température voisine de 20°C, puis ajoutée, goutte à goutte, à une solution de 1,3 g d'amino-2 N,N-diphényl acétamide et de 1,2 g de triéthylamine dans 30 cm3 de dichloro-1,2 éthane. La suspension obtenue est agitée pendant 18 heures à une température voisine de 20°C puis versée dans 60 cm3 d'eau distillée. Le produit insoluble est séparé par filtration, lavé à l'eau distillée et séché à l'air. On obtient, après recristallisation dans un mélange de diméthylformamide et d'acétonitrile (1-25 en volumes), 1 g de N-(diphénycarbamoylméthyl) indolecarboxamide-2 fondant à 218°C.

EXEMPLE 10

En opérant d'une manière analogue à celle décrite à l'exemple 9, mais à partir de 0,95 g d'acide naphtalènecarboxylique-2, de 0,7 g de dichlorure d'oxalyle, de 1,2 g d'amino-2 N,N-diphényl acétamide et de 0,8 g de triéthylamine, on obtient, après recristallisation dans l'acétonitrile, 1,2 g de N-(diphénylcarbamoylméthyl) naphtalènecarboxamide-2 fondant à 205°C.

EXEMPLE 11

En opérant d'une manière analogue à celle décrite à l'exemple 9, mais à partir de 1,3 g d'acide indolecarboxylique-2, de 1 g de dichlorure d'oxalyle et de 1,2 g d'amino-2 N,N-diphényl propionamide-(RS), on obtient, après recristallisation dans l'acétonitrile, 1,6 g de N-(diphénylcarbamoyl-1 éthyl) indolecarboxamide-2 - (RS) fondant à 242°C.

EXEMPLE 12

En opérant d'une manière analogue à celle décrite à l'exemple 1, mais à partir de 1,25 g d'(amino-2 N-phényl acétamido)-2 acétate de tert-butyle et de 0,63 g d'isocyanate de méthyl-3 phényle, on obtient, après recristallisation dans l'acétonitrile, 0,8 g de {[(méthyl-3 phényl)-3 uréido]-2 N-phényl acétamido}-2 acétate de tert-butyle fondant à 180°C.

L'(amino-2 N-phényl acétamido)-2 acétate de tert-butyle peut être préparé de la manière suivante : à une solution de 2 g de (phtalimido-2 N-phényl acétamido)-2 acétate de tert-butyle dans 20 cm3 de méthanol, on ajoute 0,25 g d'hydrate d'hydrazine. Le mélange réactionnel est agité à reflux pendant 1 heure, puis le produit insoluble est séparé par filtration. Le filtrat est concentré à sec sous pression réduite (2,7 kPa) à 30°C. Le résidu obtenu est agité avec 25 cm3 d'éther diisopropylique et le produit insoluble est séparé par filtration. Le filtrat est concentré à sec sous pression réduite (2,7 kPa) à 30°C. On obtient ainsi 0,8 g d'(amino-2 N-phényl acétamido)-2 acétate de tert-butyle sous forme d'une huile jaune.

Le (phtalimido-2 N-phényl acétamido)-2 acétate de tert-butyle peut être préparé d'une manière analogue à celle décrite à l'exemple 7 pour la préparation du phtalimido-2 N,N-diphényl propionamide-(RS), mais à partir de 8,9 g de (chloro-2 N-phényl acétamido)-2 acétate de tert-butyle et de 12,8 g de phtalimide potassé. On obtient ainsi 8,8 g de (phtalimido-2 N-phényl acétamido)-2 acétate de tert-butyle fondant à 126°C.

Le (chloro-2 N-phényl acétamido)-2 acétate de tert-butyle peut être préparé d'une manière analogue à celle décrite à l'exemple 7 pour la préparation du chloro-2 N,N-diphényl proprionamide (RS), mais à partir de 21,6 g de N-phényl glycinate de tert-butyle et de 17,8 g de chlorure de chloracétyle. On obtient ainsi 9 g de (chloro-2 N-phényl acétamido)-2 acétate de tert-butyle fondant à 86°C.

Le N-phényl glycinate de tert-butyle peut être préparé de la manière suivante : à une solution de 56 g d'aniline dans 600 cm3 de dichloro-1,2 éthane, on ajoute 58 g de bromoacétate de tert-butyle. La solution obtenue est agitée à reflux pendant 48 heures. Après refroidissement, le produit insoluble est séparé par filtration et le filtrat est lavé par 200 cm3 d'une solution aqueuse d'acide chlorhydrique 0,1N et par 3 fois 200 cm3 d'eau distillée. La phase organique est séchée sur sulfate de magnésium puis concentrée à sec sous pression réduite (2,7 kPa) à 40°C. On obtient ainsi 54 g de N-phényl glycinate de tert-butyle sous forme d'une huile brune.

EXEMPLE 13

En opérant d'une manière analogue à celle décrite à l'exemple 1, mais à partir de 0,7 g d'amino-2 N-phényl N-[oxo-2 (pyrrolidinyl-1)-2 éthyl] acétamide et de 0,36 g d'isocyanate de méthyl-3 phényle, on obtient, après recristallisation dans l'acétonitrile, 0,55 g de [(méthyl-3 phényl)-3 uréido]-2 N-phényl N-[oxo-2 (pyrrolidinyl-1)-2 éthyl] acétamide fondant à 206°C.

L'amino-2 N-phényl N-[oxo-2 (pyrrolidinyl-1)-2 éthyl] acétamide peut être préparé d'une manière analogue à celle décrite à l'exemple 7 pour la préparation de l'amino-2 N,N-diphényl propionamide-(RS), mais à partir de 1,9 g de phtalimido-2 N-phényl N-[oxo-2 (pyrrolidinyl-1)-2 éthyl] acétamide et de 0,49 g d'hydrate d'hydrazine. On obtient ainsi 0,7 g d'amino-2 N-phényl N-[oxo-2 (pyrrolidinyl-1)-2 éthyl] acétamide sous forme d'une huile jaune.

Le phtalimido-2 N-phényl N-[oxo-2 (pyrrolidinyl-1)-2 éthyl] acétamide peut être préparé de la manière suivante : à une suspension de 2 g d'acide (phtalimido-2 N-phényl acétamido)-2 acétique dans 20 cm3 de tétrahydrofuranne anhydre, on ajoute 1 g de N,N'-diimidazole-carbonyle. Le mélange est agité 2 heures à une température voisine de 25°C, puis on ajoute 0,46 g de pyrrolidine. La solution obtenue est agitée à reflux pendant 1 heure, puis concentrée à sec sous pression réduite (2,7 kPa) à 40°C. Le résidu est dissous dans 40 cm3 d'acétate d'éthyle et la solution obtenue est lavée avec 20 cm3 d'une solution aqueuse d'acide chlorhydrique N puis 2 fois 20 cm3 d'eau distillée. La phase organique est séchée sur sulfate de magnésium puis concentrée à sec sous pression réduite (2,7 kPa) à 40°C. On obtient ainsi 2 g de phtalimido-2 N-phényl N-[oxo-2 (pyrrolidinyl-1)-2 éthyl] acétamide sous forme d'une poudre blanche amorphe.

L'acide (phtalimido-2 N-phényl acétamido)-2 acétique peut être préparé de la manière suivante : à une solution de 8 g de (phtalimido-2 N-phényl acétamido)-2 acétate de tert-butyle dans 30 cm3 de dichlorométhane, on ajoute 17,9 g d'acide trifluoroacétique. La solution obtenue est agitée à reflux pendant 1 heure, puis concentrée à sec sous pression réduite (2,7 kPa) à 40°C. On obtient, après recristallisation dans l'éther diisopropylique, 5,9 g d'acide (phtalimido-2 N-phényl acétamido)-2 acétique fondant à 224°C.

EXEMPLE 14

En opérant d'une manière analogue à celle décrite à l'exemple 1, mais à partir de 1,9 g d'amino-2 N-benzyl N-phényl acétamide et de 1,1 g d'isocyanate de méthyl-3 phényle. On obtient après recristallisation dans l'acétonitrile, 2,3 g de N-benzyl N-phényl [(méthyl-3 phényl)-3 uréido]-2 acétamide fondant à 175°C.

L'amino-2 N-benzyl N-phényl acétamide peut être préparé d'une manière analogue à celle décrite à l'exemple 7 pour la préparation de l'amino-2 N,N-diphényl propionamide-(RS), mais à partir de 12,5 g de phtalimido-2 N-benzyl N-phényl acétamide et de 3,44 g d'hydrate d'hydrazine. On obtient ainsi 8,1 g d'amino-2 N-benzyl N-phényl acétamide sous forme d'une huile jaune.

Le phtalimido-2 N-benzyl N-phényl acétamide peut être préparé d'une manière analogue à celle décrite à l'exemple 7 pour la préparation de phtalimido-2 N,N-diphényl propionamide (RS), mais à partir de 10,3 g de N-benzyl chloro-2 N-phényl acétamide et de 14,8g de phtalimide potassé. On obtient ainsi 12,5 g de phtalimido-2 N-benzyl N-phényl acétamide fondant à 150°C.

Le N-benzyl chloro-2 N-phényl acétamide peut être préparé selon la méthode décrite par Y.V. SVETKIN, A.N. Minlibaeva, Zh. Obshch. Khim., 33(4), 1108 (1963) (C.A. 59, 9920a (1963).

EXEMPLE 15

Une suspension de 4,5 g d'acide [(méthyl-3 phényl)-3 uréido]-2 méthylthio-4 butanoïque - (RS) et de 3,3 g d'anilino acétate de tert-butyle dans 300 cm3 de dichloro-1,2 éthane anhydre est chauffée à reflux jusqu'à obtention d'une solution. On ajoute alors 1,16 cm3 de chlorure de thionyle en maintenant le reflux jusqu'à la fin du dégagement gazeux. Le mélange réactionnel est alors versé dans 100 cm3 d'une solution aqueuse saturée de bicarbonate de sodium et la phase organique est lavée avec 100 cm3 d'eau, séchée sur sulfate de magnésium et concentrée à sec sous pression réduite (2,7 kPa) à 40°C. Le résidu huileux obtenu est purifié par chromatographie sur 350 g de gel de silice (0,04-0,063 mm) contenus dans une colonne de 5 cm de diamètre [éluant : chlorure de méthylène-méthanol (98-2 en volumes)] en utilisant une surpression de 40 kPa d'azote et en recueillant des fractions de 100 cm3. Les fractions 12 à 28 sont réunies et concentrées à sec sous pression réduite (2,7 kPa) à 40°C. Par recristallisation du résidu ainsi obtenu dans l'oxyde de diéthyle, on obtient 2,8 g de N-{[(méthyl-3 phényl)-3 uréido]-2 méthylthio-4 butyryl} N-phényl glycinate de tert-butyle-(RS) fondant à 142°C.

L'acide [(méthyl-3 phényl)-3 uréido]-2 méthylthio-4 butanoïque-(RS) peut être préparé de la façon suivante : à une suspension de 6 g de DL-méthionine et de 3,36 g de bicarbonate de sodium dans 60 cm3 d'eau distillée, on ajoute, à une température voisine de 20°C, 5,2 g d'isocyanate de méthyl-3 phényle et le mélange est agité pendant 16 heures à une température voisine de 20°C. Le produit insoluble est ensuite séparé par filtration et le filtrat acidifié à pH 1 à l'aide d'une solution aqueuse 4N d'acide chlorhydrique. Le solide obtenu est séparé par filtration, lavé à l'eau et séché à l'air. On obtient ainsi 9,6 g d'acide [(méthyl-3 phényl)-3 uréido]-2 méthylthio-4 butanoïque-(RS) fondant à 143°C.

EXEMPLE 16

En opérant comme à l'exemple 15, mais à partir de 4,8 g d'acide [(méthyl-3 phényl)-3 uréido]-2 phényl-3 propanoïque-(RS) et de 3,3 g d'anilino acétate de tert-butyle dans 300 cm3 de dichloro-1,2 éthane anhydre et de 1,16 cm3 de chlorure de thionyle. On obtient en recritallisant successivement dans l'oxyde de diéthyle puis dans l'isopropanol, 3,2 g de N-{[(méthyl-3 phényl)-3 uréido]-2 phényl-3 propionyl} N-phényl glycinate de tert-butyle-(RS) fondant à 198°C.

L'acide [(méthyl-3 phényl)-3 uréido]-2 phényl-3 propanoïque-(RS) peut être préparé d'une manière analogue à celle décrite à l'exemple 15 pour la préparation de l'acide [(méthyl-3 phényl)-3 uréido]-2 méthylthio-4 butanoïque-(RS), mais à partir de 6,6 g de DL-phénylalanine, de 3,36 g de bicarbonate de sodium, de 100 cm3 d'eau et de 5,3 g d'isocyanate de méthyl-3 phényle. On obtient ainsi 9,7 g d'acide [(méthyl-3 phényl)-3 uréido]-2 phényl-3 propanoïque-(RS) fondant à 182°C.

EXEMPLE 17

A une solution de 3,6 g de N,N'-diimidazole-carbonyle dans 80 cm3 de dichloro-1,2 éthane anhydre, on ajoute une solution de 4,53 g d'amino-2 N,N-diphényl acétamide dans 50 cm3 de dichloro-1,2 éthane anhydre. La solution obtenue est agitée pendant 2 heures à une température voisine de 20°C puis on ajoute 6,4 g de dichloro-2,3 aniline et le mélange est agité pendant 20 heures à reflux. Après refroidissement, le mélange réactionnel est lavé par 3 fois 75 cm3 d'eau. La phase organique est séchée sur sulfate de magnésium et concentrée à sec sous pression réduite (2,7 kPa). Le résidu huileux obtenu est purifié par chromatographie sur 250 g de gel de silice (0,04-0,063 mm) contenus dans une colonne de 4,2 cm de diamètre (éluant : chlorure de méthylène-méthanol (98-2 en volumes)] en utilisant une surpression de 40 kPa d'azote et en recueillant des fractions de 20 cm3. Les fractions 11 à 20 sont réunies et concentrées à sec sous pression réduite (2,7 kPa) à 40°C et

le résidu obtenu est recristallisé dans le dioxanne. On obtient ainsi 1,4 g de [(dichloro-2,3 phényl)-3 uréido] N,N-diphényl acétamide fondant à 210°C.

EXEMPLE 18

En opérant d'une manière analogue à celle décrite à l'exemple 15, mais à partir de 1,56 g d'acide [(méthyl-3 phényl)-3 uréido]-2 acétique, de 1,45 g d'anilino-2 propionate d'éthyle-(RS) dans 30 cm3 de dichloro-1,2 éthane anhydre et de 0,89 g de chlorure de thionyle, on obtient, après recristallisation dans un mélange d'acétate d'éthyle et d'oxyde d'isopropyle (12-1 en volumes), 1 g de {[(méthyl-3 phényl)-3 uréido]-2 N-phényl acétamido}-2 propionate d'éthyle (RS) fondant à 113-8°C.

L'anilino-2 propionate d'éthyle-(RS) peut être préparé selon la méthode décrite par M. JULIA et G. TCHERNOFF, Bull. Soc. Chim. France, 661 (1958).

EXEMPLE 19

En opérant d'une manière analogue à celle décrite à l'exemple 15, mais à partir de 0,95 g d'acide [(méthyl-3 phényl)-3 uréido]-2 acétique, de 1,15 g d'anilino-2 phénylacétate d'éthyle-(RS) dans 15 cm3 de dichloro-1,2 éthane anhydre et de 0,53 g de chlorure de thionyle, on obtient, après recristallisation dans l'acétonitrile, 1,1 g de {[(méthyl-3 phényl)-3 uréido]-2 N-phényl acétamido}-2 phénylacétate d'éthyle (RS) fondant à 120°C.

L'anilino-2 phénylacétate d'éthyle-(RS) peut être préparé selon la méthode décrite par M. JULIA et G. TCHERNOFF, Bull. Soc. Chim. France, 661 (1958).

EXEMPLE 20

A une solution de 2 g d'amino-2 N-(chloro-3 phényl) N-phényl acétamide dans 30 cm3 de tétrahydrofuranne anhydre, on ajoute à une température voisine de 20°C, 1,06 g d'isocyanate de phényle. La suspension obtenue est agitée pendant 3 heures à une température voisine de 20°C puis concentrée à sec sous pression réduite (2,7 kPa) à 40°C. On obtient ainsi, après recristallisation du résidu dans l'acétonitrile 2,3 g de N-(chloro-3 phényl) [(méthyl-3 phényl)-3 uréido]-2 N-phényl acétamide fondant à 184°C.

L'amino-2 N-(chloro-3 phényl)N-phényl acétamide peut être préparé de la manière suivante : à une solution de 5,2 g de [phtalimido-2 N-(chloro-3 phényl) N-phényl acétamide dans 40 $cm^3$de méthanol, on ajoute 1,33 g d'hydrate d'hydrazine. Le mélange réactionnel est agité à reflux pendant 3 heures puis refroidi à une température voisine de 20°C et additionné d'une solution aqueuse d'acide chlorhydrique 5 N jusqu'à un pH voisin de 1. Le produit insoluble est séparé par filtration et éliminé ; le filtrat est concentré à sec sous pression réduite (2,7 kPa) à 40°C. L'huile résiduelle est dissoute dans 50 $cm^3$ d'acétate d'éthyle et la solution obtenue est additionnée de 50$cm^3$ d'eau distillée puis d'une solution aqueuse d'hydroxyde de sodium 1 N jusqu'à un pH voisin de 8. La phase aqueuse est séparée par décantation puis réextraite par 2 fois 30 $cm^3$ d'acétate d'éthyle. Les phases organiques sont réunies, lavées par 2 fois 10 $cm^3$ d'eau distillée, séchées sur sulfate de magnésium, filtrées puis concentrées à sec sous pression réduite (2,7 kPa) à 40°C. On obtient ainsi 1 g d'amino-2 N-(chloro-3 phényl) N-phényl acétamide sous forme d'une huile jaune utilisée telle quelle dans les synthèses ultérieures.

Le phtalimido-2 N-(chloro-3 phényl) N-phényl acétamide peut être préparé de la manière suivante : à une solution de 6,1 g de chloro-3 diphénylamine dans 50 $cm^3$ de dichloro-1,2 éthane, maintenue sous atmosphère d'argon, on ajoute 4,2 g de triéthylamine puis goutte à goutte, à une température voisine de 20°C, une solution de 9,7 g de chlorure de phtalimido-2 acétyle dans 20 $cm^3$ de dichloro-1,2 éthane. La solution obtenue est agitée pendant 4 heures à une température voisine de 20°C, puis pendant 1 heure à 60°C, refroidie à une température voisine de 20°C et additionnée de 50 $cm^3$ d'eau distillée. La phase aqueuse est séparée par décantation puis réextraite par 2 fois 30 $cm^3$ de dichloro-1,2 éthane. Les phases organiques sont réunies, lavées par 2 fois 20 $cm^3$ d'eau distillée, séchées sur sulfate de magnésium, filtrées puis concentrées à sec sous pression réduite (2,7 kPa) à 40°C. On obtient ainsi, après recristallisation dans un mélange d'acétate d'éthyle et d'oxyde de diisopropyle (70-30 en volumes), 5,4 g de phtalimido-2 N-(chloro-3 phényl) N-phényl acétamide fondant à 185°C.

Le chlorure de phtalimido-2 acétyle peut être préparé selon la méthode décrite par W. GRASSMANN et al., Chem. Ber., 83, 244 (1950).

EXEMPLE 21

En opérant d'une manière analogue à celle décrite à l'exemple 20 mais à partir de 2,9 g d'amino-2 N-(méthoxy-4 phényl) N-phényl acétamide et de 1,65 g d'isocyanate de phényle, on obtient après recristallisation

dans l'acétate d'éthyle, 1,78 g de N-(méthoxy-4 phényl) N-phényl [(méthyl-3 phényl)-3 uréido]-2 acétamide fondant à 182°C.

L'amino-2 N-(méthoxy-4 phényl) N-phényl acétamide peut être préparé d'une manière analogue à celle décrite à l'exemple 20 pour la préparation de l'amino-2 N-(chloro-3 phényl) N-phényl acétamide, mais à partir de 5 g de phtalimido-2 N-(méthoxy-4 phényl) N-phényl-acétamide et de 1,3 g d'hydrate d'hydrazine. On obtient ainsi 3,1 g d'amino-2 N-(méthoxy-4 phényl) N-phényl acétamide sous forme d'une huile utilisée telle quelle dans les synthèses ultérieures.

Le phtalimido-2 N-(méthoxy-4 phényl) N-phényl acétamide peut être préparé d'une manière analogue à celle décrite à l'exemple 20 pour la préparation du phtalimido-2 N-(chloro-3 phényl) N-phényl acétamide, mais à partir de 6 g de méthoxy-4 diphénylamine, de 4 g de triéthylamine et de 8,7 g de chlorure de phtalimido-2 acétyle. On obtient ainsi, après recristallisation dans l'acétate d'éthyle, 5,1 g de phtalimido-2 N-(méthoxy-4 phényl) N-phényl acétamide fondant à 192°C.

La méthoxy-4 diphénylamine peut être préparée selon la méthode décrite par R. WILLSTATTER et H. KUBLI, Chem. Ber., 42, 4135-4151 (1909).

EXEMPLE 22

En opérant d'une manière analogue à celle décrite à l'exemple 1 mais à partir de 1,7 g d'(amino-2 N-phényl-acétamido)-2 N,N-dipropyl-acétamide et de 0,77 g d'isocyanate de méthyl-3 phényle, on obtient après recristallisation dans l'acétonitrile, 1,1 g de {[(méthyl-3 phényl)-3 uréido]-2 N-phényl acétamido}-2 N,N-dipropyl-acétamide fondant à 182°C.

L'(amino-2 N-phényl-acétamido)-2 N,N-dipropyl-acétamide peut être préparé d'une manière analogue à celle décrite à l'exemple 7 pour la préparation de l'amino-2 N,N-diphényl propionamide mais à partir de 2,7 g de N,N-dipropyl (phtalimido-2 N-phényl-acétamido)-2 acétamide et de 1,2 g d'hydrate d'hydrazine. On obtient ainsi, 1,7 g d'(amino-2 N-phényl acétamido)-2 N,N-dipropyl-acétamide sous forme d'une huile utilisée telle quelle dans les synthèses ultérieures.

Le N,N-dipropyl (phtalimido-2 N-phényl acétamido)-2 acétamide peut être préparé de la manière suivante : à une suspension de 4 g d'acide (phtalimido-2 N-phényl acétamido)-2 acétique dans 50 $cm^3$ de dichloro-1,2 éthane, on ajoute 1,7 g de dichlorure d'oxalyle puis une goutte de diméthylformamide. Le mélange est agité 2 heures à une température voisine de 20°C puis on ajoute 2,4 g de dipropylamine en solution dans 20 $cm^3$ de dichloro-1,2 éthane. La solution obtenue est agitée 2 heures à une température voisine de 25°C puis lavée par 2 fois 30 $cm^3$ d'eau distillée, séchée sur sulfate de magnésium et concentrée à sec sous pression réduite (2,7 kPa) à 40°C. On obtient après recristallisation dans l'oxyde de diisopropyle 2,7 g de N,N-dipropyl (phtalimido-2 N-phényl acétamido)-2 acétamide fondant à 130°C.

L'acide (phtalimido-2 N-phényl-acétamido)-2 acétique peut être préparé de la manière suivante : à une solution de 8 g de (phtalimido-2 N-phényl acétamido)-2 acétate de tert-butyle dans 30 $cm^3$ de dichlorométhane, on ajoute 17,9 g d'acide trifluoroacétique. La solution obtenue est agitée à reflux pendant 1 heure, puis concentrée à sec sous pression réduite (2,7 kPa) à 40°C. On obtient, après recristallisation dans l'éther diisopropylique, 5,9 g d'acide (phtalimido-2 N-phényl-acétamido)-2 acétique fondant à 224°C.

Le (phtalimido-2 N-phényl acétamido)-2 acétate de tert-butyle peut être préparé de la manière suivante : à une solution de 207 g de N-phényl glycinate de tert-butyle dans 500 $cm^3$ de dichloro-1,2 éthane, on ajoute 92,4 g d'hydrogénocarbonate de sodium. La suspension est agitée à une température voisine de 5°C et on ajoute une solution de 223 g de chlorure de phtalimido-2 acétyle dans 1100 $cm^3$ de dichloro-1,2 éthane. Le mélange réactionnel est agité à reflux pendant 4 heures. Après séparation du produit insoluble par filtration, le filtrat est lavé par 300 $cm^3$ d'eau distillée, séché sur sulfate de magnésium et concentré à sec sous pression réduite (2,7 kPa) à 40°C. On obtient, après recristallisation dans l'acétonitrile 236 g de (phtalimido-2 N-phényl acétamido)-2 acétate de tert-butyle fondant à 128°C.

Le N-phényl glycinate de tert-butyle peut être préparé de la manière suivante : à une solution de 56 g d'aniline dans 600 $cm^3$ de dichloro-1,2 éthane, on ajoute 58 g de bromoacétate de tert-butyle et la solution obtenue est agitée à reflux pendant 48 heures. Après refroidissement, le produit insoluble est séparé par filtration et le filtrat est lavé par 200 $cm^3$ d'une solution aqueuse d'acide chlorhydrique 0,1N et par 3 fois 200 cm3 d'eau distillée. La phase organique est séchée sur sulfate de magnésium puis concentrée à sec sous pression réduite (2,7 kPa) à 40°C. On obtient ainsi 54 g de N-phényl glycinate de tert-butyle sous forme d'une huile utilisée telle quelle dans les synthèses ultérieures.

Le chlorure de phtalimido-2 acétyle peut être préparé selon la méthode décrite par W. GRASSMANN et E. SCHULTE-UEBLING, Chem. Ber 83, 244, (1950).

EXEMPLE 23

A une solution de 1,78 g de N,N'-carbonyldiimidazole dans 50 $cm^3$ de dichloro-1,2 éthane anhydre, on ajoute une solution de 2,64 g d'amino-2 N-phényl acétamido)-2 acétate de tert-butyle dans 25 $cm^3$ de dichloro-1,2 éthane anhydre. La solution obtenue est agitée pendant 1 heure à une température voisine de 20°C puis on ajoute 1,4 g de méthylthio-3 aniline et le mélange est chauffé pendant 4 heures à reflux. Après refroidissement, on ajoute 100 $cm^3$ de dichlorométhane et le mélange réactionnel est lavé par 3 fois 75 $cm^3$ d'eau, 3 fois 75 $cm^3$ d'une solution aqueuse d'acide chlorhydrique 0,1 N puis 3 fois 75 $cm^3$ d'eau. La phase organique est séchée sur sulfate de magnésium et concentrée à sec sous pression réduite (2,7 kPa). Le résidu obtenu est purifié par chromatographie sur 250 g de gel de silice (0,04-0,063 mm) contenus dans une colonne de 3 cm de diamètre [éluant : dichlorométhane-méthanol (97,5-2,5 en volumes)] en utilisant une surpression de 40 kPa d'azote et en recueillant des fractions de 20 $cm^3$. Les fractions 8 à 14 sont réunies et concentrées à sec sous pression réduite (2,7 kPa) à 40°C et le résidu obtenu est recristallisé dans l'acétonitrile. On obtient ainsi 1,6 g de N-{[(méthylthio-3 phényl)-3 uréido]-2 acétyl} N-phényl glycinate de tert butyle fondant à 139°C.

EXEMPLE 24

On opère d'une manière analogue à celle décrite à l'exemple 23, mais à partir de 0,9 g de N,N'-diimidazolecarbonyle dans 25 $cm^3$ de dichloro-1,2 éthane anhydre, de 1,35 g d'(amino-2 N-phényl acétamido)-2 acétate de tert-butyle dans 10 $cm^3$ de dichloro-1,2 éthane anhydre et de 0,62 g de méthoxy-3 aniline. On obtient, après recristallisation dans l'acétonitrile, 0,7 g de N-{[(méthoxy-3 phényl)-3 uréido]-2 acétyl} N-phényl glycinate de tert-butyle fondant à 136°C.

EXEMPLE 25

A une solution de 1,2 g d'(amino-2 N-phényl acétamido]-2 acétate de tert-butyle dans 35 $cm^3$ de dichloro-1,2 éthane agitée à une température voisine de 25°C, on ajoute 0,7 g de triéthylamine, puis 1,2 g de chlorure d'indolecarbonyle-2 en solution dans 35 $cm^3$ de dichloro-1,2 éthane. Le mélange réactionnel est agité pendant 18 heures à une température voisine de 25°C. On ajoute alors 250 $cm^3$ de dichlorométhane puis 125 $cm^3$ d'une solution aqueuse saturée d'hydrogénocarbonate de sodium. La phase organique est lavée par 2 fois 125 $cm^3$ d'eau distillée, séchée sur sulfate de magnésium et concentrée à sec sous pression réduite (2,7 kPa) à 40°C. On obtient, après recristallisation dans l'acétonitrile, 1 g de N-(N-tert-butoxycarbonylméthyl phénylcarbamoylméthyl) indolecarboxamide-2 fondant à 203°C.

Le chlorure d'indolecarbonyle-2 peut être préparé de la manière suivante : à une suspension de 1,85 g d'acide indolecarboxylique-2 dans 40 $cm^3$ d'oxyde de diéthyle anhydre à une température voisine de 5°C, on ajoute 0,1 $cm^3$ de diméthylformamide, puis 1,5 g de dichlorure d'oxalyle en solution dans 10 $cm^3$ d'oxyde de diéthyle anhydre. Le mélange réactionnel est agité à une température voisine de 25°C pendant 2 heures. La phase éthérée est concentrée à sec sous pression réduite (2,7 kPa) à 30°C. On obtient ainsi 1,8 g de chlorure d'indolecarbonyle-2 fondant à 120°C.

EXEMPLE 26

A une solution de 1,91 g d'acide dichloro-3,4 benzoïque dans 50 $cm^3$ de dichloro-1,2 éthane anhydre, on ajoute 5 mg de diméthylamino-4 pyridine puis, 1,78 g de N,N-carbonyldiimidazole. La solution obtenue est agitée pendant 2 heures à une température voisine de 20°C puis on ajoute 2,64 g d'(amino-2 N-phényl acétamido)-2 acétate de tert-butyle et le mélange est agité pendant 3 heures à une température voisine de 20°C. Le mélange réactionnel est alors lavé par 3 fois 75 $cm^3$ d'eau, la phase organique est séparée, séchée sur sulfate de magnésium et concentrée à sec sous pression réduite (2,7 kPa). Le résidu obtenu est purifié par chromatographie sur 250 g de gel de silice (0,04-0,063 mm) contenus dans une colonne de 4,2 cm de diamètre [éluant : dichlorométhane-méthanol (98-2 en volumes)] en utilisant une surpression de 40 kPa d'azote et en recueillant des fractions de 20 $cm^3$. Les fractions 7 à 15 sont réunies et concentrées à sec sous pression réduite (2,7 kPa) à 40°C et le résidu est recristallisé dans un mélange de cyclohexane et de propanol-2 (80-20 en volumes). On obtient ainsi 1,5 g de N-[N-(dichloro-3,4 benzoyl) glycyl] N-phényl glycinate de tert-butyle fondant à 95°C.

EXEMPLE 27

On opère d'une manière analogue à celle décrite à l'exemple 26 , mais à partir de 1,72 9 d'acide naphtoïque-2, de 5 mg de diméthylamino-4 pyridine, de 1,78 g de N,N'-diimidazolecabonyle et de 2,64 g d'(amino-2

N-phényl acétamido)-2 acétate de tert-butyle. On obtient ainsi, après recristallisation dans un mélange d'heptane et de propanol-2 (70-30 en volumes), 2,3 g de N-[N-(naphtoyl-2) glycyl] N-phényl glycinate de tert-butyle fondant à 108°C.

EXEMPLE 28

Une suspension de 2,9 g d'anilino-2 acétate de cyclopropyle et de 2,9 g d'acide [(méthyl-3 phényl)-3 uréido]-2 acétique dans 50 $cm^3$ de dichloro-1-2 éthane anhydre est chauffée à reflux. On ajoute alors 1,0 $cm^3$ de chlorure de thionyle en maintenant le reflux jusqu'à la fin du dégagement gazeux. Le mélange réactionnel est alors versé dans 30 $cm^3$ d'une solution aqueuse saturée d'hydrogénocarbonate de sodium puis on ajoute 50 $cm^3$ de chlorure de méthylène. La phase organique est lavée par 50 $cm^3$ d'eau distillée, séchée sur sulfate de magnésium et concentrée à sec sous pression réduite (2,7 kPa) à 40°C. On obtient, après recristallisation dans l'acétonitrile, 1,6 g de {[(méthyl-3 phényl)-3 uréido]-2 N-phényl acétamido}-2 acétate de cyclopropylméthyle fondant à 158°C.

L'anilino-2 acétate de cyclopropylméthyle peut être préparé de la manière suivante : à une solution de 3,7 g d'aniline dans 50 $cm^3$ d'acétonitrile on ajoute 4 g de bromoacétate de cyclopropylméthyle et le mélange est agité à reflux pendant 3 heures. Le produit insoluble est séparé par filtration et le filtrat est concentré à sec sous pression réduite (2,7 kPa) à 40°C. Le produit obtenu est purifié par chromatographie sur 50 g de gel de silice (0,065-0,200 mm) contenus dans une colonne de 2 cm de diamètre [éluant : cyclohexane-acétate d'éthyle (70-30 en volumes)] en recueillant des fractions de 20 $cm^3$. Les fractions 3 à 5 sont réunies et concentrées à sec sous pression réduite (2,7 kPa) à 40°C. On obtient ainsi 2,9 g d'anilino-2 acétate de cyclopropylméthyle sous forme d'une huile utilisée telle quelle dans les synthèses ultérieures.

Le bromoacétate de cyclopropylméthyle peut être préparé de la manière suivante : à une solution de 2,3 g de cyclopropylméthanol et de 3,3 g de triéthylamine dans 40 $cm^3$ d'éther diétylique maintenue à une température voisine de 5°C, on ajoute en 20 minutes 6 g de bromure de bromoacétyle puis le mélange est agité pendant 2 heures à une température voisine de 25°C. le produit insoluble est séparé par filtration et le filtrat est lavé successivement par 20 $cm^3$ d'une solution aqueuse d'acide chlorhydrique 4N, 20 $cm^3$ d'une solution aqueuse saturée d'hydrogénocarbonate de sodium, 25 $cm^3$ d'eau distillée, puis séché sur sulfate de magnésium et concentré à sec sous pression réduite (2,7 kPa) à 30°C. On obtient ainsi 4 g de bromoacétate de cyclopropylméthyle sous forme d'une huile utilisée telle quelle dans les synthèses ultérieures.

L'acide [(méthyl-3 phényl)-3 uréido]-2 acétique peut être préparé de la manière suivante : à une solution de 30 g de glycine et de 53 g d'hydrogénocarbonate de sodium dans 600 $cm^3$ d'eau distillée, on ajoute en 15 minutes 53 g d'isocyanate de méthyl-3 phényle. Le mélange réactionnel est agité 4 heures à une température voisine de 25°C puis lavé par 200 $cm^3$ d'acétate d'éthyle et acidifié à pH 1 avec 200 $cm^3$ d'une solution d'acide chlorhydrique 4N. Le produit obtenu est séparé par filtration, lavé à l'eau et séché à l'air. On obtient ainsi 72 g d'acide [(méthyl-3 phényl)-3 uréido]-2 acétique fondant à 208°C.

EXEMPLE 29

On opère d'une manière analogue à celle décrite à l'exemple 28 mais à partir de 1,5 g d'anilino-2 phényl-2 acétate de tert-butyle (RS), de 1,1 g d'acide [(méthyl-3 phényl)-3 uréido]-2 acétique et de 0,63 g de chlorure de thionyle. Le produit obtenu est purifié par chromatographie sur 30 g de gel de silice (0,063-0,200 mm) contenus dans une colonne de 2,2 cm de diamètre [éluant : chlorure de méthylène-acétate d'éthyle (97-3 en volumes)] en recueillant des fractions de 20 $cm^3$. Les fractions 12 à 35 sont réunies et concentrées à sec sous pression réduite (2,7 kPa) à 40°C. On obtient, après recristallisation dans un mélange d'oxyde de diisopropyle et d'hexane (65-35 en volumes), 0,85 g de {[(méthyl-3 phényl)-3 uréido]-2 N-phényl acétamido}-2 phénylacétate de tert-butyle (RS) fondant à 131°C.

L'anilino-2 phényl-2 acétate de tert-butyle (RS) peut être préparé d'une manière analogue à celle décrite à l'exemple 28 pour la préparation de l'anilino-2 acétate de cyclopropylméthyle, mais à partir de 6,7 g d'α-bromo phénylacétate de tert-butyle et de 3,7 g d'aniline. Le produit obtenu est purifié par chromatographie sur 25 g de gel de silice (0,063-0,200mm) contenus dans une colonne de 2,7 cm de diamètre (éluant : éther de pétrole-acétate d'éthyle (85-15 en volumes)] en recueillant des fractions de 20 $cm^3$. Les fractions 6 à 10 sont réunies et concentrées à sec sous pression réduite (2,7 kPa) à 40°C. On obtient ainsi 1,6 g d'anilino-2 phényl-2 acétate de tert-butyle-(RS) fondant à 94°C.

L'α-bromo phénylacétate de tert-butyle peut être préparé d'une manière analogue à celle décrite à l'exemple 28 pour la préparation du bromoacétate de cyclopropylméthyle , mais à partir de 21,8 g de méthyl-2 propanol-2, de 21,4 g de N,N-diméthylaniline (en remplacement de la triéthylamine) et de 16 g de chlorure d'α-bromo phénylacétyle. On obtient ainsi 8,1 g d'α-bromo phénylacétate de tert-butyle sous forme d'une huile

jaune.

Le chlorure d'α-bromo phénylacétyle peut être préparé selon la méthode décrite par V.L. NARAYANAN et C.F. MARTIN, J. Med. Chem., 9, 616 (1966).

EXEMPLE 30

On opère d'une manière analogue à celle décrite à l'exemple 28 mais à partir de 1,8 g d'anilino-2 acétate de sec-butyle-(RS), de 1,8 g d'acide [(méthyl-3 phényl)-3 uréido]-2 acétique et de 0,6 $cm^3$ de chlorure de thionyle. Le résidu huileux obtenu est purifié par chromatographie sur 30 g de gel de silice (0,063-0,200 mm) contenus dans une colonne de 1,5 cm de diamètre [éluant : dichlorométhane-méthanol (70-30 en volumes)] en recueillant des fractions de 20 $cm^3$. Les fractions 10 à 12 sont réunies et concentrées à sec sous pression réduite (2,7 kPa) à 40°C. On obtient, après recristallisation dans l'oxyde d'isopropyle, 0,7 g de {[(méthyl-3 phényl)-3 uréido]-2 N-phényl acétamido}-2 acétate de sec-butyle-(RS) fondant à 136°C.

L'anilino-2 acétate de sec-butyle-(RS) peut être préparé d'une manière anlogue à celle décrite à l'exemple 28 pour la préparation de l'anilino-2 acétate de cyclopropylméthyle, mais à partir de 2,7 g de bromoacétate de sec-butyle-(RS) et de 2,7 g d'aniline. Le produit obtenu est purifié par chromatographie sur 50 g de gel de silice (0,063-0,200 mm) contenus dans une colonne de 2 cm de diamètre [éluant : cyclohexane-acétate d'éthyle (70-30 en volumes)] en recueillant des fractions de 20 $cm^3$. Les fractions 2 à 5 sont réunies et concentrées à sec sous pression réduite (2,7 kPa) à 40°C. On obtient ainsi 1,8 g d'anilino-2 acétate de sec-butyle-(RS) sous forme d'une huile utilisée telle quelle dans les synthèses ultérieures.

Le bromoacétate de sec-butyle-(RS) peut être préparé d'une manière analogue à celle décrite à l'exemple 28 pour la préparation du bromoacétate de cyclopropylméthyle mais à partir de 2,2 g de butanol-2-(RS), de 3,2 g de triéthylamine et de 6 g de bromure de bromoacétyle. On obtient ainsi 2,7 g de bromoacétate de sec-butyle-(RS) sous forme d'une huile utilisée telle quelle dans les synthèses ultérieures.

EXEMPLE 31

En opérant d'une manière analogue à celle décrite à l'exemple 28, mais à partir de 2,2 g d'anilino-2 acétate d'éthyle, de 2,6 g d'acide [(méthyl-3 phényl)-3 uréido]-2 acétique et de 1,50 g de chlorure de thionyle, on obtient après recristallisation dans l'isopropanol, 2 g de {[(méthyl-3 phényl)-3 uréido]-2 N-phényl acétamido}-2 acétate d'éthyle fondant à 181°C.

L'anilino-2 acétate d'éthyle peut être préparé d'une manière analogue à celle décrite à l'exemple 28 pour la préparation de l'anilino-2 acétate de cyclopropylméthyle, mais à partir de 10,8 g d'aniline et de 9,3 g de bromoacétate d'éthyle. On obtient ainsi 7 g d'anilino-2 acétate d'éthyle fondant à environ 40°C.

EXEMPLE 32

On opère d'une manière analogue à celle décrite à l'exemple 28, mais à partir de 3,6 g d'anilino-2 acétate de benzyle, de 3,1 g d'acide [(méthyl-3 phényl)-3 uréido]-2 acétique et de 1,65 g de chlorure de thionyle. Le produit obtenu est purifié par chromatographie sur 60 g de gel de silice (0,063-0,200 mm) contenus dans une colonne de 2 cm de diamètre [éluant : cyclohexane-acétate d'éthyle (50-50 en volumes)] en recueillant des fractions de 20 $cm^3$. Les fractions 5 à 9 sont réunies et concentrées à sec sous pression réduite (2,7 kPa) à 40°C. On obtient après recristallisation dans l'acétonitrile, 1,4 g de {[(méthyl-3 phényl)-3 uréido]-2 N-phényl acétamido}-2 acétate de benzyle fondant à 140°C.

L'anilino-2 acétate de benzyle peut être préparé d'une manière analogue à celle décrite à l'exemple 28 pour la préparation de l'anilino-2 acétate de cyclopropylméthyle, mais à partir de 3,9 g d'aniline et de 4,9 g de bromoacétate de benzyle. Le produit obtenu est purifié par chromatographie sur 50 g de gel de silice (0,063-0,200 mm) contenus dans une colonne de 1,5 cm de diamètre [éluant : cyclohexane-acétate d'éthyle (70-30 en volumes)] en recueillant des fractions de 20 $cm^3$. Les fractions 3 à 6 sont réunies et concentrées à sec sous pression réduite (2,7 kPa) à 40°C. On obtient 3,8 g d'anilino-2 acétate de benzyle sous forme d'une huile utilisée telle quelle dans les synthèses ultérieures.

Le bromoacétate de benzyle peut être préparé d'une manière analogue à celle décrite à l'exemple 28 pour la préparation du bromoacétate de cyclopropylméthyle mais à partir de 2,7 g d'alcool benzylique, de 2,8 g de triéthylamine et de 5 g de bromure de bromoacétyle. On obtient ainsi 4,9 g de bromoacétate de benzyle sous forme d'une huile utilisée telle quelle dans les synthèses ultérieures.

EXEMPLE 33

On opère d'une manière analogue à celle décrite à l'exemple 28 , mais à partir de 8,6 g d'anilino-2 acétate de cyclohexyle, de 7,7 g d'acide [(méthyl-3 phényl)-3 uréido]-2 acétique et de 2,7 $cm^3$ de chlorure de thionyle. Le produit obtenu est purifié par chromatographie sur 150 g de gel de silice (0,063-0,200 mm) contenus dans une colonne de 3,5 cm de diamètre [éluant : chlorure de méthylène-méthanol (99-1 en volumes)] en recueillant des fractions de 20 $cm^3$. Les fractions 6 à 18 sont réunies et concentrées à sec sous pression réduite (2,7 kPa) à 40°C. On obtient après recristallisation dans l'isopropanol, 2,3 g de {[(méthyl-3 phényl)-3 uréido]-2 N-phényl acétamido}-2 acétate de cyclohexyle fondant à 176°C.

L'anilino-2 acétate de cyclohexyle peut être préparé d'une manière analogue à celle décrite à l'exemple 28 pour la préparation de l'anilino-2 acétate de cyclopropylméthyle, mais à partir de 11,5 g de bromoacétate de cyclohexyle et de 9,7 g d'aniline. Le produit obtenu est purifié par chromatographie sur 180 g de gel de silice (0,063-0,200 mm) contenus dans une colonne de 3,5 cm de diamètre [éluant : cyclohexane-acétate d'éthyle (70-30 en volumes)] en recueillant des fractions de 20 $cm^3$. Les fractions 2 à 10 sont réunies et concentrées à sec sous pression réduite (2,7 kPa) à 40°C. On obtient ainsi 8,6 g d'anilino-2 acétate de cyclohexyle fondant à environ 50°C.

Le bromoacétate de cyclohéxyle peut être préparé d'une manière analogue à celle décrite à l'exemple 28 pour la préparation du bromoacétate de cyclopropylméthyle mais à partir de 9 g de cyclohexanol, de 9,9 g de triéthylamine et de 18 g de bromure de bromoacétyle. Le produit obtenu est purifié par chromatographie sur 200 g de gel de silice (0,063-0,200 mm) contenus dans une colonne de 3,5 cm de diamètre [éluant : cyclohexane-acétate d'éthyle (80-20 en volumes)] en recueillant des fractions de 20 $cm^3$. Les fractions 3 à 12 sont réunies et concentrées à sec sous pression réduite (2,7 kPa) à 40°C. On obtient ainsi 11,5 g de bromoacétate de cyclohexyle sous forme d'une huile utilisée telle quelle dans les synthèses ultérieures.

EXEMPLE 34

Le {[(méthyl-3 phényl)-3 uréido]-2 N-phényl acétamido}-2 acétate de phényle peut être préparé de la manière suivante : à une suspension de 1 g d'acide {[(méthyl-3 phényl)-3 uréido]-2 N-phényl acétamido}-2 acétique et de 0,28 g de phénol dans 60 $cm^3$ de chlorure de méthylène, on ajoute 0,6 g de triéthylamine et 1,28 g d'hexafluorophosphonate de benzotriazolyl-1 oxy-tris (diméthylamino) phosphonium. La solution obtenue est agitée pendant 2 heures à une température voisine de 25°C, puis versée dans 50 $cm^3$ d'une solution aqueuse saturée de chlorure de sodium. La phase organique est séparée et la phase aqueuse est extraite avec 3 fois 80 $cm^3$ d'acétate d'éthyle. Les phases organiques réunies sont lavées successivement par 2 fois 30 $cm^3$ d'une solution aqueuse d'acide chlorhydrique 2N, 2 fois 30 $cm^3$ d'une solution aqueuse saturée d'hydrogénocarbonate de sodium, 50 $cm^3$ d'une solution aqueuse saturée de chlorure de sodium, séchées sur sulfate de magnésium et concentrées à sec sous pression réduite (2,7 kPa) à 40°C. On obtient, après recristallisation dans l'acétonitrile, 0,7 g de {[(méthyl-3 phényl)-3 uréido]-2 N-phényl acétamido}-2 acétate de phényle fondant à 206°C.

L'acide {[(méthyl-3 phényl)-3 uréido]-2 N-phényl-acétamido}-2 acétique peut être préparé de la manière suivante : à une solution de 3,3 g de {[(méthyl-3 phényl)-3 uréido]-2 N-phényl acétamido}-2 acétate de tert-butyle dans 25 $cm^3$ de dichlorométane, on ajoute 7 g d'acide trifluoroacétique. La solution obtenue est agitée au reflux pendant 4 heures, puis concentrée à sec sous pression réduite (2,7 kPa) à 40°C. On obtient, après recristallisation dans l'éther diisopropylique, 2 g d'acide {[(méthyl-3 phényl)-3 uréido]-2 N-phényl-acétamido}-2 acétique fondant à 188°C.

EXEMPLE 35

Une suspension de 5 g de N-(méthyl-3 phénylcarbamoyl) phénylglycine-(RS) et de 3,6 g d'anilino acétate de tert-butyle dans 300 $cm^3$ de dichloro-1,2 éthane anhydre est chauffée à reflux en agitant. On ajoute ensuite 1,3 $cm^3$ de chlorure de thionyle en maintenant le reflux pendant 10 minutes. Après refroidissement le mélange réactionnel est versé dans 100 $cm^3$ d'une solution aqueuse saturée de bicarbonate de sodium et la phase organique est lavée avec 100 cm3 d'eau, séchée sur sulfate de magnésium et concentrée à sec sous pression réduite (2,7 kPa) à 40°C. Le résidu obtenu est purifié par chromatographie sur 250 g de gel de silice (0,04-0,063mm) contenus dans une colonne de 5 cm de diamètre [éluant : dichlorométhane-méthanol (98-2 en volumes)] en utilisant une surpression de 40 kPa d'azote et en recueillant des fractions de 100 $cm^3$. Les fractions 16 à 21 sont réunies et concentrées à sec sous pression réduite (2,7 kPa) à 40°C et le résidu obtenu est recristallisé dans l'oxyde de diisopropyle. On obtient ainsi 4,3 g de N-{[(méthyl-3 phényl)-3 uréido]-2 phényl-2 acétyl} N-phényl glycinate de tert-butyle-(RS) fondant à 178°C.

La N-(méthyl-3 phénylcarbamoyl) phénylglycine-(RS) peut être préparée de la façon suivante : à une suspension de 6,05 g de phénylglycine-(RS) et de 3,36 g d'hydrogénocabonate de sodium dans 100 $cm^3$ d'eau, on ajoute en 1 minute, 5,14 $cm^3$ d'isocyanate de méthyl-3 phényle. Le mélange est agité pendant 20 heures à une température voisine de 20°C puis est extrait par 3 fois 75 $cm^3$ d'acétate d'éthyle. La phase organique est lavée par 2 fois 50 $cm^3$ d'eau, les extraits aqueux réunis sont acidifiés à pH 1 par une solution aqueuse 4N d'acide chlorhydrique. Le précipité obtenu est séparé par filtration, lavé à l'eau et séché sous pression réduite (0,07 kPa) à 40°C. On obtient ainsi 5 g de N-(méthyl-3 phénylcarbamoyl) phénylglycine-(RS) fondant à 198°C.

## EXEMPLE 36

On opère comme à l'exemple 35, mais à partir de 5,7 g de N-(méthyl-3 phénylcarbamoyl) phénylglycine-(RS), de 3,38 g de diphénylamine dans 350 $cm^3$ de dichloro-1,2 éthane anhydre et de 1,45 $cm^3$ de chlorure de thionyle. Après recristallisation dans l'oxyde de diéthyle, on obtient 2 g de [(méthyl-3 phényl)-3 uréido]-2 phényl-2 N,N-diphényl acétamide-(RS) fondant à 181°C.

## EXEMPLE 37

On opère comme à l'exemple 35, mais à partir de 4,8 g de N-(méthyl-3 phénylcabamoyl) ß-alanine, de 4,5 g d'anilino acétate de tert-butyle et de 1,6 $cm^3$ de chlorure de thionyle. Après recristallisation dans l'oxyde de diéthyle, on obtient 4,1 g de {[(méthyl-3 phényl)-3 uréido]-3 N-phényl-propionamido}-2 acétate de tert-butyle-(RS) fondant à 142°C.

La N-(méthyl-3 phénylcarbamoyl)-β-alanine peut être préparée de manière analogue à celle décrite à l'exemple 35 pour la préparation de la N-(méthyl-3 phénylcarbamoyl) phénylglycine-(RS) mais à partir de 3,56 g de β-alanine, de 3,36 g d'hydrogénocarbonate de sodium et de 5,14 $cm^3$ d'isocyanate de méthyl-3 phényle. On obtient ainsi 4,87 g de N-(méthyl-3 phénylcarbamoyl) β-alanine fondant à 166°C.

## EXEMPLE 38

On opère comme à l'exemple 35, mais à partir de 5 g de N-(méthyl-3 phénylcarbamoyl)-valine-(RS), de 4,1 g d'anilino acétate de tert-butyle et de 1,45 $cm^3$ de chlorure de thionyle. Après 2 recristallisations successives, d'abord dans l'oxyde de diisopropyle puis dans le cyclohexane, on obtient 3,5 g de {[(méthyl-3 phényl)-3 uréido]-2 méthyl-3 N-phényl butyramino}-2 acétate de tert-butyle-(RS) fondant à 154°C.

La N-(méthyl-3 phénylcarbamoyl)-valine-(RS) peut être préparée de manière analogue à celle décrite à l'exemple 35 pour la préparation de la N-(méthyl-3 phénylcarbamoyl)-phénylglycine-(RS), mais à partir de 4,7 g de valine-(RS), de 3,36 g d'hydrogénocarbonate de sodium et de 5,14 $cm^3$ d'isocyanate de méthyl-3 phényle. On obtient ainsi 6,6 g de N-(méthyl-3 phénylcarbamoyl) valine-(RS) fondant à 170°C.

La présente invention concerne également les médicaments constitués par au moins un composé de formule (I) à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple

par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, des pommades, des lotions, des collyres, des collutoires, des gouttes nasales ou des aérosols.

En thérapeutique humaine, les composés selon l'invention sont particulièrement utiles dans le traitement et la prévention des désordres liés à la CCK au niveau du système nerveux et de l'appareil gastrointestinal. Ces composés peuvent donc être utilisés dans le traitement et la prévention des psychoses, des troubles anxieux de la maladie de Parkinson, de la diskinésie tardive, du syndrome du colon irritable, de la pancréatite aigüe, des ulcères, des désordres de la motilité intestinale, de certaines tumeurs de l'oesophage inférieur, du colon et de l'intestin, comme potentialisateur de l'activité des médicaments analgésiques narcotiques et non narcotiques et comme régulateur de l'appétit.

Les doses dépendent de l'effet recherché, de la durée du traitement et de la voie d'administration utilisée ; elles sont généralement comprises entre 0,05 g et 1 g par jour par voie orale pour un adulte avec des doses unitaires allant de 10 mg à 500 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

EXEMPLE A

On prépare, selon la technique habituelle, des gélules dosées à 50 mg de produit actif ayant la composition suivante :

- N,N-diphényl [(méthyl-3 phényl)-3 uréido]-2 acétamide .......... 50 mg
- cellulose .......... 18 mg
- lactose .......... 55 mg
- silice colloïdale .......... 1 mg
- carboxyméthylamidon sodique .......... 10 mg
- talc .......... 10 mg
- stéarate de magnésium .......... 1 mg

EXEMPLE B

On prépare, selon la technique habituelle, des comprimés dosés à 50 mg de produit actif ayant la composition suivante :

| | |
|---|---|
| - N,N-diphényl (phényl-3 uréido)-2 acétamide ........... | 50 mg |
| - lactose ................................................ | 104 mg |
| - cellulose .............................................. | 40 mg |
| - polyvidone ............................................. | 10 mg |
| - carboxyméthylamidon sodique ........................... | 22 mg |
| - talc ................................................... | 10 mg |
| - stéarate de magnésium ................................. | 2 mg |
| - silice colloïdale ..................................... | 2 mg |
| - mélange d'hydroxyméthylcellulose, glycérine, oxyde de titane (72-3,5-24,5) .................... q. s. p. | 1 comprimé pelliculé terminé à 245 mg |

EXEMPLE C

On prépare une solution injectable contenant 10 mg de produit actif ayant la composition suivante :

| | |
|---|---|
| - {[(méthyl-3 phényl)-3 uréido]-2 N-phényl acétamido}-2 acétate de tert-butyle .................. | 10 mg |
| - acide benzoïque ........................................ | 80 mg |
| - alcool benzylique ...................................... | 0,06 cm3 |
| - benzoate de sodium ..................................... | 80 mg |
| - éthanol à 95 % ......................................... | 0,4 cm3 |
| - hydroxyde de sodium .................................... | 24 mg |
| - propylène glycol ....................................... | 1,6 cm3 |
| - eau ..................................... q. s. p. | 4 cm3 |

## Revendications

### Revendications pour les Etats contractants suivants : AT, BE, CH, DE, DK, FR, GB, IT, LI, LU, NL, SE

1. Composés de formule :

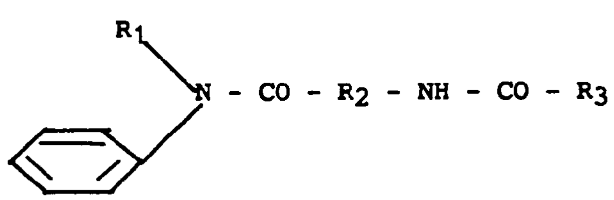

(I)

dans laquelle
- $R_1$ représente :
  - un radical phényle ou phényle substitué par un radical alkyle, alcoxy, cyano ou par un atome d'halogène,
  - une chaîne $-CH(R_8)-COOR_4$ dans laquelle $R_4$ représente un atome d'hydrogène ou un radical alkyle, cycloalkyle, cycloalkylalkyle, phénylalkyle ou phényle et $R_8$ représente un atome d'hydrogène ou un radical alkyle ou phényle,
- une chaîne $-CH_2-CO-NR_5R_6$ dans laquelle $R_5$ et $R_6$ identiques ou différents représentent un radical alkyle ou forment avec l'atome d'azote auquel ils sont rattachés un radical pyrrolidinyl-1 éventuellement substitué par un radical alkyle ou
- un radical phénylalkyle,

- $R_2$ représente un radical méthylène, éthylène ou $-CH(R_7)-$ dans lequel $R_7$ représente un radical alkyle, benzyle, alkylthioalkyle dont les portions alkyle contiennent 1 ou 2 atomes de carbone, ou phényle,
- $R_3$ représente un radical naphtyl-1 ou -2, indolyl-2 ou -3 ou phénylamino dont le noyau phényle est éventuellement substitué par un radical alkyle, alcoxy, nitro, hydroxy ou alkylthio ou par 1 ou 2 atomes d'halogène,

étant entendu que lorsque $R_3$ représente un radical naphtyl-1 ou -2 ou indolyl-2 ou -3, $R_7$ ne peut pas représenter un radical benzyle ou alkylthioalkyle ou phényle et que, sauf mention contraire, les radicaux alkyle et alcoxy et les portions alkyle et alcoxy contiennent 1 à 4 atomes de carbone en chaîne droite ou ramifiée, ainsi que lorsque $R_2$ représente un radical $-CH(R_7)-$, leurs racémiques et leurs stéroisomères.

**2.** Composés de formule (I) selon la revendication 1 pour lesquels
- $R_1$ représente
  - un radical phényle éventuellement substitué par un radical alcoxy ou par un atome d'halogène,
  - une chaîne $-CH(R_5)-COOR_4$ dans laquelle $R_4$ représente un radical alkyle ou cycloalkylalkyle et $R_5$ représente un atome d'hydrogène ou un radical phényle,
  - $R_2$ et $R_3$ ont les mêmes significations que dans la revendication 1.

**3.** Procédé de préparation des composés de formule (I) selon la revendication 1, pour lesquels
- $R_2$ représente un radical méthylène, éthylène ou $-CH(R_7)-$ dans lequel $R_7$ représente un radical alkyle,
- $R_3$ représente un radical phénylamino dont le noyau phényle est éventuellement substitué par un radical alkyle, alcoxy, nitro, alkylthio ou par 1 ou 2 atomes d'halogène et
- $R_1$ a les mêmes définitions que dans la revendication 1, caractérisé ce que l'on fait réagir un dérivé de formule :

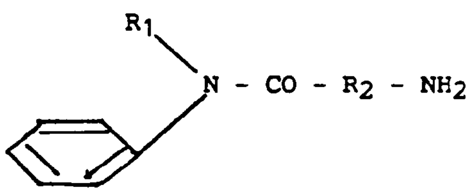

(II)

dans laquelle $R_2$ et $R_1$ ont les mêmes significations que ci-dessus, sur un phénylisocyanate dont le noyau phényle est éventuellement substitué par un radical alkyle, alcoxy, nitro, alkylthio ou par 1 ou 2 atomes d'halogène, puis isole le produit.

**4.** Procédé de préparation des composés de formule (I) selon la revendication 1, pour lesquels
- $R_2$ représente un radical méthylène, éthylène ou $-CH(R_7)-$ dans lequel $R_7$ représente un radical alkyle,
- $R_3$ représente un radical phénylamino dont le noyau phényle est éventuellement substitué par un radical alkyle, alcoxy, nitro, alkylthio, hydroxy ou par 1 ou 2 atomes d'halogène et
- $R_1$ a les mêmes définitions que dans la revendication 1, caractérisé en ce que l'on fait réagir un composé de formule :

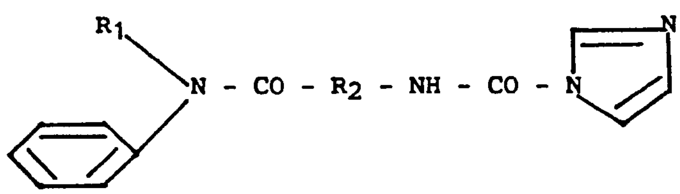

(X)

dans laquelle $R_1$ et $R_2$ ont les mêmes significations que ci-dessus, sur une aniline éventuellement

substituée par un radical alkyle, alcoxy, alkylthio, nitro, hydroxy ou par 1 ou 2 atomes d'halogène, puis isole le produit.

**5.** Procédé de préparation des composés de formule (I) selon la revendication 1, pour lesquels

- $R_2$ représente un radical méthylène, éthylène ou -CH($R_7$)- dans lequel $R_7$ représente un radical alkyle,
- $R_3$ représente un radical naphtyl-1 ou -2 ou indolyl-2 ou -3 et
- $R_1$ a les mêmes significations que dans la revendication 1, caractérisé en ce que l'on fait réagir un dérivé de formule :

HOOC-$R_3$ (XI)

dans laquelle $R_3$ représente un radical naphtyl-1 ou -2 ou indolyl-2 ou -3 ou un dérivé réactif de cet acide sur un dérivé de formule :

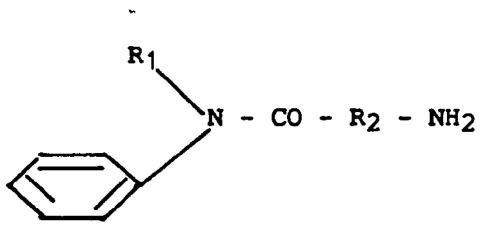

(II)

dans laquelle $R_2$ et $R_1$ ont les mêmes significations que ci-dessus puis isole le produit.

**6.** Procédé de préparation des composés de formule (I) selon la revendication 1 pour lesquels

- $R_3$ représente un radical phénylamino dont le noyau phényle est éventuellement substitué par un radical alkyle, alcoxy, nitro, alkylthio ou par 1 ou 2 atomes d'halogène et
- $R_1$ et $R_2$ ont les mêmes significations que dans la revendication 1, à l'exception pour $R_1$ de représenter une chaîne - CH($R_8$)-COO$R_4$ dans laquelle $R_4$ représente un atome d'hydrogène ou une chaîne -$CH_2$-CO-N$R_5R_6$ caractérisé en ce que l'on fait réagir une amine de formule :

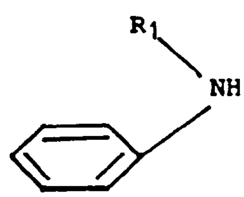

(V)

dans laquelle $R_1$ a les mêmes significations que ci-dessus sur un dérivé de formule :

HOOC - $R_2$ - NH - CO - $R_3$ (XII)

dans laquelle $R_2$ et $R_3$ ont les mêmes significations que ci-dessus puis isole le produit.

**7.** Procédé de préparation des composés de formule (I) selon la revendication 1 pour lesquels

- $R_3$ représente un radical phénylamino dont le noyau phényle est éventuellement substitué par un radical alkyle, alcoxy, nitro, alkylthio ou par 1 ou 2 atomes d'halogène,
- $R_2$ a les mêmes significations que dans la revendication 1 et,
- $R_1$ représente une chaîne - CH($R_8$)-COO$R_4$ dans laquelle $R_4$ représente un atome d'hydrogène,

caractérisé en ce que l'on hydrolyse un dérivé de formule (I) correspondant pour lequel $R_1$ représente une chaîne - CH($R_5$)-COO$R_4$ dans laquelle $R_4$ représente un radical alkyle puis isole le produit.

**8.** Procédé de préparation des composés de formule (I) selon la revendication 1 pour lesquels

- $R_3$ représente un radical phénylamino dont le noyau phényle est éventuellement substitué par un radical alkyle, alcoxy, nitro, alkylthio ou par 1 ou 2 atomes d'halogène,
- $R_2$ a les mêmes significations que dans la revendication 1 et
- $R_1$ représente une chaîne - $CH_2$-CO-N$R_5R_5$

caractérisé en ce que l'on fait réagir un acide en formule (I) correspondant pour lequel $R_1$ représente une

chaîne - $CH(R_8)$-$COOR_4$ dans laquelle $R_4$ représente un atome d'hydrogène ou un dérivé réactif de cet acide, avec une amine de forumule :

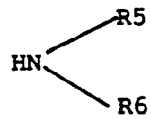

(VIII)

dans laquelle $R_5$ et $R_6$ ont les mêmes significations que dans la revendication 1, puis isole le produit.

**9.** Procédé de préparation des composés de formule (I) selon la revendication 1 pour lesquels
- $R_3$ représente un radical phénylamino dont le noyau phényle est substitué par un radical hydroxy et
- $R_1$ et $R_2$ ont les mêmes significations que dans la revendication 1, caractérisé en ce que l'on désalkyle le composé de formule (I) correspondant pour lequel $R_3$ représente un radical phénylamino dont le noyau phényle est substitué par un radical alcoxy puis isole de produit.

**10.** Procédé de préparation des composés de formule (I) selon la revendication 1 pour lesquels $R_1$ représente une chaîne -$CH(R_8)$ - $COOR_4$ dans laquelle $R_4$ représente un radical phényle caractérisé en ce que l'on fait réagir le phénol sur un composé de formule (I) correspondant pour lequel $R_4$ représente un atome d'hydrogène, puis isole le produit.

**11.** Médicaments caractérisés en ce qu'ils contiennent en tant que principe actif au moins un composé de formule (I) selon la revendication 1, à l'état pur ou sous forme d'une association avec un ou plusieurs diluants ou adjuvants compatibles et pharmaceutiquement acceptables.

**12.** Médicaments selon la revendication 11 pour le traitement ou la prévention des désordres liés à la CCK au niveau du système nerveux et de l'appareil gastrointestinal.

**Revendication pour les Etats contractants suivants : ES, GR**

**1.** Procédé de préparation des composés de formule :

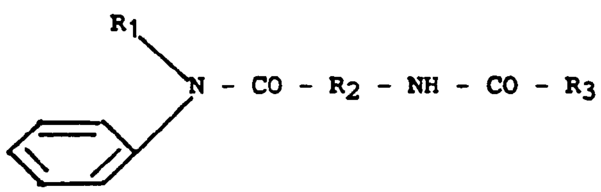

(I)

dans laquelle
- $R_1$ représente :
  - un radical phényle ou phényle substitué par un radical alkyle, alcoxy, cyano ou par un atome d'halogène,
  - une chaîne -$CH(R_6)$-$COOR_4$ dans laquelle $R_4$ représente un atome d'hydrogène ou un radical alkyle, cycloalkyle, cycloalkylalkyle, phénylalkyle ou phényle et $R_8$ représente un atome d'hydrogène ou un radical alkyle ou phényle,
  - une chaîne -$CH_2$-CO-$NR_6R_6$ dans laquelle $R_6$ et $R_6$ identiques ou différents représentent un radical alkyle ou forment avec l'atome d'azote auquel ils sont rattachés un radical pyrrolidinyl-1 éventuellement substitué par un radical alkyle ou
  - un radical phénylalkyle,
- $R_2$ représente un radical méthylène, éthylène ou -$CH(R_7)$- dans lequel $R_7$ représente un radical alkyle, benzyle, alkylthioalkyle dont les portions alkyle contiennent 1 ou 2 atomes de carbone ou phényle,
- $R_3$ représente un radical naphtyl-1 ou -2, indolyl-2 ou -3 ou phénylamino dont le noyau phényle est éventuellement substitué par un radical alkyle, alcoxy, nitro, hydroxy ou alkylthio ou par 1 ou 2 atomes d'halogène,

étant entendu que lorsque $R_3$ représente un radical naphtyl -1 ou -2 ou indolyl-2 ou -3, $R_7$ ne peut pas représenter un radical benzyle, alkylthioalkyle ou phényle et que, sauf mention contraire, les radicaux alkyle et alcoxy et les portions alkyle et alcoxy contiennent 1 à 4 atomes de carbone en chaîne droite ou ramifiée,
ainsi que lorsque $R_2$ représente un radical -CH($R_7$)-, les racémiques et leurs stéroisomères, caractérisé en ce que :

A. pour la préparation des composés de formule (I) pour lesquels $R_2$ représente un radical méthylène, éthylène ou -CH($R_7$) - dans lequel $R_7$ représente un radical alkyle, $R_3$ représente un radical phénylamino dont le noyau phényle est éventuellement substitué par un radical alkyle, alcoxy, nitro, alkylthio ou par un ou deux atomes d'halogène, on fait réagir un dérivé de formule :

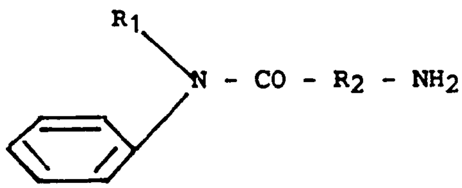

(II)

dans laquelle $R_1$ et $R_2$ ont les mêmes significations que précédemment sur un phénylisocyanate dont le noyau phényle est éventuellement substitué par un radical alkyle, alcoxy, nitro, alkylthio ou par un ou deux atomes d'halogène, puis isole le produit,

B. pour la préparation des composés de formule (I) pour lesquels $R_2$ représente un radical méthylène, éthylène ou - CH ($R_7$) dans lequel $R_7$ représente un radical alkyle, $R_3$ représente un radical phénylamino dont le noyau phényle est éventuellement substitué par un radical alkyle, alcoxy, nitro, alkylthio, hydroxy ou par un ou deux atomes d'halogène, on fait réagir un composé de formule :

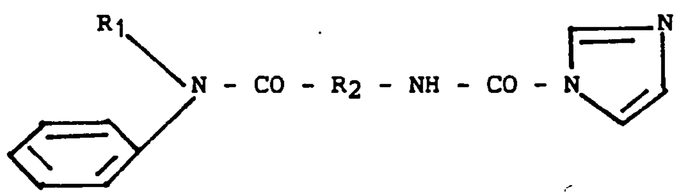

(X)

dans laquelle $R_1$ et $R_2$ ont les mêmes significations que précédemment sur une aniline éventuellement substituée par un radical alkyle, alcoxy, alkylthio, nitro, hydroxy ou par un ou deux atomes d'halogène, puis isole le produit.

C. pour la préparation des composés de formule (I) pour lesquels $R_2$ représente un radical méthylène, éthylène ou - CH ($R_7$) - dans laquelle $R_7$ représente un radical alkyle et $R_3$ représente un radical naphtyl - 1 ou -2 ou indolyl -2 ou -3, on fait réagir un dérivé de formule :

$$HOOC\text{-}R_3 \qquad (XI)$$

dans laquelle $R_3$ représente un radical naphtyl - 1 ou - 2 ou indolyl - 2 ou - 3 ou un dérivé réactif de cet acide sur un dérivé de formule :

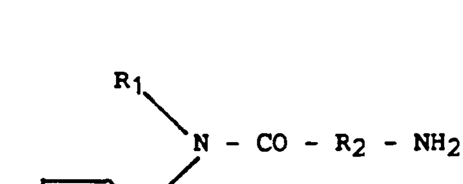

(II)

dans laquelle $R_1$ et $R_2$ ont les mêmes significations que précédemment, puis isole le produit.

D. pour la préparation des composés de formule (I) pour lesquels $R_3$ représente un radical phénylamino dont le noyau phényle est éventuellement substitué par un radical alkyle, alcoxy, nitro, alkylthio ou par un ou deux atomes d'halogène et $R_1$ et $R_2$ ont les mêmes significations que ci-dessus à l'exception pour $R_1$ de représenter une chaîne - CH ($R_8$) - COOR$_4$ dans laquelle $R_4$ représente un atome d'hydrogène ou une chaîne - $CH_2$ - CO - NR$_5$R$_8$, on fait réagir une amine de formule :

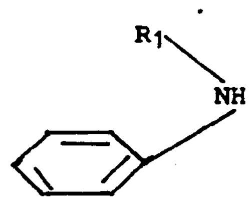

(V)

dans laquelle $R_1$ a les mêmes significations que précédemment sur un dérivé de formule :

HOOC - $R_2$ - NH - CO - $R_3$ (XII)

dans laquelle $R_2$ et $R_3$ ont les mêmes significations que précédemment et isole le produit,

E. pour la préparation des composés de formule (I) pour lesquels $R_3$ représente un radical phénylamino dont le noyau phényle est éventuellement substitué par un radical alkyle, alcoxy, nitro, alkylthio ou par un ou deux atomes d'halogène et $R_1$ représente une chaîne - CH ($R_8$) - $COOR_4$ dans laquelle $R_4$ représente un atome d'hydrogène, on hydrolyse un dérivé de formule (I) correspondant pour lequel $R_1$ représente une chaîne - CH ($R_8$) - $COOR_4$ dans laquelle $R_4$ représente un radical alkyle, puis isole le produit,

F. pour la préparation des composés de formule (I) pour lesquels $R_3$ représente un radical phénylamino dont le noyau phényle est éventuellement substitué par un radical alkyle, alcoxy, nitro, alkylthio ou par un ou deux atomes d'halogène et $R_1$ représente une chaîne - $CH_2$ - CO - $NR_8R_8$, on fait réagir un acide de formule (I) correspondant pour lequel $R_1$ représente une chaîne - CH ($R_8$) - $COOR_4$ dans laquelle $R_4$ représente un atome d'hydrogène ou un dérivé réactif de cet acide, avec une amine de formule :

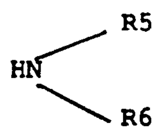

(VIII)

dans laquelle $R_5$ et $R_6$ ont les mêmes significations que ci-dessus, puis isole le produit.

G. pour la préparation des composés de formule (I) pour lesquels $R_3$ représente un radical phénylamino dont le noyau phényle est substitué par un radical hydroxy on désalkyle un composé de formule (I) correspondant pour lequel $R_3$ représente un radical phénylamino dont le noyau phényle est substitué par un radical alcoxy, puis isole le produit,

H. pour la préparation des composés de formule (I) pour lesquels $R_1$ représente une chaîne - CH ($R_8$) - $COOR_4$ dans laquelle $R_4$ représente un radical phényle, on fait réagir le phénol sur un composé de formule (I) correspondant pour lequel $R_4$ représente un atome d'hydrogène puis isole le produit.

## Claims

### Claims for the following Contracting States : AT, BE, CH, DE, DK, FR, GB, IT, LI, LU, NL, SE

**1.** Compounds of formula:

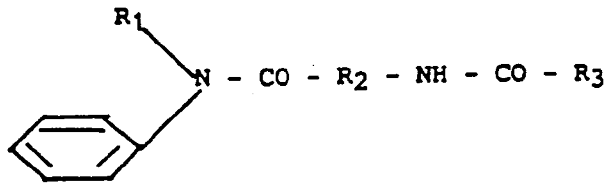

(I)

in which

- $R_1$ represents:
  - a phenyl radical or a phenyl radical substituted by an alkyl, alkoxy or cyano radical or by a halogen atom,
  - a chain -CH($R_8$)-COOR, in which $R_4$ represents a hydrogen atom or an alkyl, cycloalkyl, cycloalkylalkyl, phenylalkyl or phenyl radical and $R_8$ represents a hydrogen atom or an alkyl or phenyl radical,

- a chain $-CH_2-CO-NR_5R_6$ in which $R_8$ and $R_6$, which are identical or different, represent an alkyl radical or form, with the nitrogen atom to which they are attached, a 1-pyrrolidinyl radical optionally substituted by an alkyl radical or
- a phenylalkyl radical,

- $R_2$ represents a methylene or ethylene radical or a radical $-CH(R_7)-$ in which $R_7$ represents an alkyl, benzyl or alkylthioalkyl radical in which the alkyl portions contain 1 or 2 carbon atoms, or a phenyl radical,
- $R_3$ represents a 1- or 2-naphthyl, 2- or 3-indolyl or phenylamino radical in which the phenyl ring is optionally substituted by an alkyl, alkoxy, nitro, hydroxyl or alkylthio radical or by 1 or 2 halogen atoms, it being understood that when $R_3$ represents a 1- or 2-naphthyl or 2- or 3-indolyl radical, $R_7$ cannot represent a benzyl or alkylthioalkyl or phenyl radical and that, except when otherwise stated, the alkyl and alkoxy radicals and the alkyl and alkoxy portions contain 1 to 4 carbon atoms in a straight or branched chain, and when $R_2$ represents a radical $-CH(R_7)-$, their racemates and their stereoisomers.

2. Compounds of formula (I) according to claim 1, for which
- $R_1$ represents
  - a phenyl radical optionally substituted by an alkoxy radical or by a halogen atom,
  - a chain $-CH(R_8)-COOR_4$ in which $R_4$ represents an alkyl or cycloalkylalkyl radical and $R_8$ represents a hydrogen atom or a phenyl radical,
  - $R_2$ and $R_3$ have the same meanings as in claim 1.

3. Process for the preparation of the compounds of formula (I) according to claim 1, for which
  - $R_2$ represents a methylene or ethylene radical or a radical $-CH(R_7)-$ in which $R_7$ represents an alkyl radical,
  - $R_3$ represents a phenylamino radical in which the phenyl ring is optionally substituted by an alkyl, alkoxy, nitro or alkylthio radical or by 1 or 2 halogen atoms and
  - $R_1$ has the same definitions as in claim 1,

characterised in that a derivative of formula:

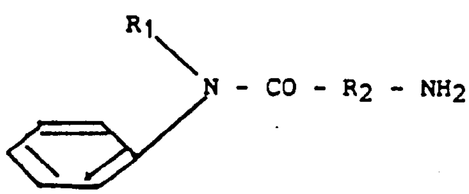

(II)

in which $R_2$ and $R_1$ have the same meanings as above, is reacted with a phenylisocyanate in which the phenyl ring is optionally substituted by an alkyl, alkoxy, nitro or alkylthio radical or by 1 or 2 halogen atoms, and then the product is isolated.

4. Process for the preparation of compounds of formula (I) according to claim 1, for which
  - $R_2$ represents a methylene or ethylene radical or a radical $-CH(R_7)-$ in which $R_7$ represents an alkyl radical,
  - $R_3$ represents a phenylamino radical in which the phenyl ring is optionally substituted by an alkyl, alkoxy, nitro, alkylthio or hydroxyl radical or by 1 or 2 halogen atoms and
  - $R_1$ has the same definitions as in claim 1,

characterised in that a compound of formula:

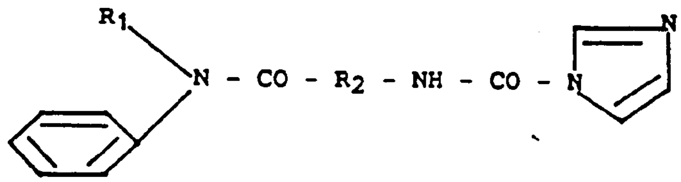

(X)

in which $R_1$ and $R_2$ have the same meanings as above, is reacted with an aniline optionally substituted by an alkyl, alkoxy, alkylthio, nitro or hydroxyl radical or by 1 or 2 halogen atoms, and then the product is isolated.

**5.** Process for the preparation of the compounds of formula (I) according to claim 1, for which

- $R_2$ represents a methylene or ethylene radical or a radical -CH($R_7$)- in which $R_7$ represents an alkyl radical,
- $R_3$ represents a 1- or 2-naphthyl or 2- or 3-indolyl radical and
- $R_1$ has the same meanings as in claim 1,

characterised in that a derivative of formula:

HOOC-$R_3$ (XI)

in which $R_3$ represents a 1- or 2-naphthyl or 2- or 3-indolyl radical, or a reactive derivative of this acid is reacted with a derivative of formula:

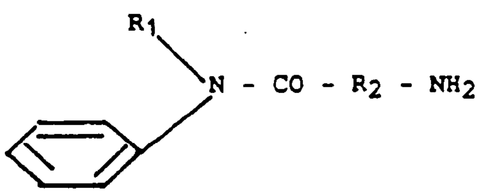

(II)

in which $R_2$ and $R_1$ have the same meanings as above, and then the product is isolated.

**6.** Process for the preparation of the compounds of formula (I) according to claim 1, for which

- $R_3$ represents a phenylamino radical in which the phenyl ring is optionally substituted by an alkyl, alkoxy, nitro or alkylthio radical or by 1 or 2 halogen atoms and
- $R_1$ and $R_2$ have the same meanings as in claim 1, except for $R_1$ representing a chain -CH($R_8$)-COOR$_4$ in which $R_4$ represents a hydrogen atom or a chain -CH$_2$-CO-NR$_5$R$_5$, characterised in that an amine of formula:

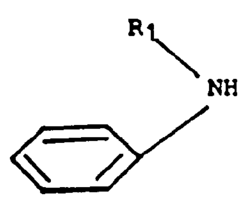

(V)

in which $R_1$ has the same meanings as above, is reacted with a derivative of formula:

HOOC-$R_2$-NH-CO-$R_3$ (XII)

in which $R_2$ and $R_3$ have the same meanings as above, and then the product is isolated.

**7.** Process for the preparation of the compounds of formula (I) according to claim 1, for which

- $R_3$ represents a phenylamino radical in which the phenyl ring is optionally substituted by an alkyl, alkoxy, nitro or alkylthio radical or by 1 or 2 halogen atoms,
- $R_2$ has the same meanings as in claim 1 and,
- $R_1$ represents a chain -CH($R_5$)-COOR$_4$ in which $R_4$ represents a hydrogen atom, characterised in that a derivative of corresponding formula (I), for which $R_1$ represents a chain -CH($R_5$)-COOR$_4$ in which $R_4$ represents an alkyl radical, is hydrolysed and then the product is isolated.

**8.** Process for the preparation of the compounds of formula (I) according to claim 1, for which

- $R_3$ represents a phenylamino radical in which the phenyl ring is optionally substituted by an alkyl, alkoxy, nitro or alkylthio radical or by 1 or 2 halogen atoms,
- $R_2$ has the same meanings as in claim 1 and
- $R_1$ represents a chain $-CH_2-CO-NR_5R_6$,

characterised in that an acid of corresponding formula (I), for which $R_1$ represents a chain $-CH(R_8)-COOR_4$ in which $R_4$ represents a hydrogen atom, or a reactive derivative of this acid is reacted with an amine of formula:

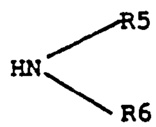

(VIII)

in which $R_5$ and $R_5$ have the same meanings as in claim 1, and then the product is isolated.

**9.** Process for the preparation of the compounds of formula (I) according to claim 1, for which

- $R_3$ represents a phenylamino radical in which the phenyl ring is substituted by a hydroxyl radical and
- $R_1$ and $R_2$ have the same meanings as in claim 1,

characterised in that the compound of corresponding formula (I), for which $R_3$ represents a phenylamino radical in which the phenyl ring is substituted by an alkoxy radical, is dealkylated and then the product is isolated.

**10.** Process for the preparation of the compounds of formula (I) according to claim 1, for which $R_1$ represents a chain $-CH(R_8)-COOR_4$ in which $R_4$ represents a phenyl radical, characterised in that the phenol is reacted with a compound of corresponding formula (I) for which $R_4$ represents a hydrogen atom and then the product is isolated.

**11.** Medicaments, characterised in that they contain as active principle at least one compound of formula (I) according to claim 1 in the pure state or in the form of a combination with one or a number of compatible and pharmaceutically acceptable diluents or adjuvants.

**12.** Medicaments according to claim 11 for the treatment or prevention of disorders linked to CCK in the nervous system and in the gastrointestinal system.

**Claims for the following Contracting States : ES, GR**

**1.** Process for the preparation of the compounds of formula:

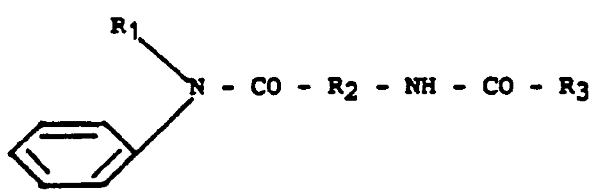

(I)

in which

- $R_1$ represents:
  - a phenyl radical or a phenyl radical substituted by an alkyl, alkoxy or cyano radical or by a halogen atom,
  - a chain $-CH(R_8)-COOR_4$ in which $R_4$ represents a hydrogen atom or an alkyl, cycloalkyl, cycloalkylalkyl, phenylalkyl or phenyl radical and $R_5$ represents a hydrogen atom or an alkyl or phenyl radical,
  - a chain $-CH_2-CO-NR_5R_6$ in which $R_5$ and $R_6$, which are identical or different, represent an alkyl radical or form, with the nitrogen atom to which they are attached, a 1-pyrrolidinyl radical optionally substituted by an alkyl radical or

- a phenylalkyl radical,

- $R_2$ represents a methylene or ethylene radical or a radical -CH($R_7$)- in which $R_7$ represents an alkyl, benzyl or alkylthioalkyl radical in which the alkyl portions contain 1 or 2 carbon atoms, or a phenyl radical,
- $R_3$ represents a 1- or 2-naphthyl, 2- or 3-indolyl or phenylamino radical in which the phenyl ring is optionally substituted by an alkyl, alkoxy, nitro, hydroxyl or alkylthio radical or by 1 or 2 halogen atoms, it being understood that when $R_3$ represents a 1- or 2-naphthyl or 2- or 3-indolyl radical, $R_7$ cannot represent a benzyl, alkylthioalkyl or phenyl radical and that, except when otherwise stated, the alkyl and alkoxy radicals and the alkyl and alkoxy portions contain 1 to 4 carbon atoms in a straight or branched chain, and when $R_2$ represents a radical -CH($R_7$)-, their racemates and their stereoisomers, characterised in that:

A. for the preparation of the compounds of formula (I) for which $R_2$ represents a methylene or ethylene radical or a radical -CH($R_7$)- in which $R_7$ represents an alkyl radical and $R_3$ represents a phenylamino radical in which the phenyl ring is optionally substituted by an alkyl, alkoxy, nitro or alkylthio radical or by one or two halogen atoms, a derivative of formula:

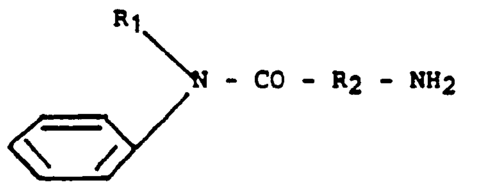

(II)

in which $R_1$ and $R_2$ have the same meanings as above, is reacted with a phenyl isocyanate in which the phenyl ring is optionally substituted by an alkyl, alkoxy, nitro or alkylthio radical or by one or two halogen atoms, and then the product is isolated,

B. for the preparation of the compounds of formula (I) for which $R_2$ represents a methylene or ethylene radical or a radical -CH($R_7$) in which $R_7$ represents an alkyl radical and $R_3$ represents a phenylamino radical in which the phenyl ring is optionally substituted by an alkyl, alkoxy, nitro, alkylthio or hydroxyl radical or by one or two halogen atoms, a compound of formula:

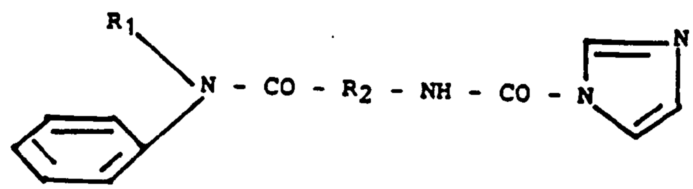

(X)

in which $R_1$ and $R_2$ have the same meanings as above, is reacted with an aniline optionally substituted by an alkyl, alkoxy, alkylthio, nitro or hydroxyl radical or by one or two halogen atoms, and then the product is isolated.

C. for the preparation of the compounds of formula (I) for which $R_2$ represents a methylene or ethylene radical or a radical -CH($R_7$)- in which $R_7$ represents an alkyl radical and $R_3$ represents a 1- or 2-naphthyl or 2- or 3-indolyl radical, a derivative of formula:

$$HOOC\text{-}R_3 \qquad (XI)$$

in which $R_3$ represents a 1- or 2-naphthyl or 2- or 3-indolyl radical, or a reactive derivative of this acid is reacted with a derivative of formula:

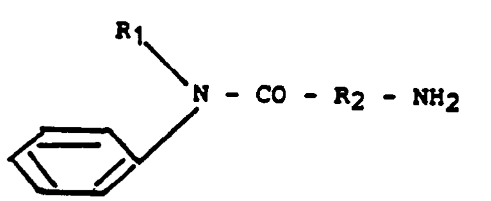

(II)

in which $R_1$ and $R_2$ have the same meanings as above, and then the product is isolated.
D. for the preparation of compounds of formula (I) for which $R_3$ represents a phenylamino radical in which the phenyl ring is optionally substituted by an alkyl, alkoxy, nitro or alkylthio radical or by one or two halogen atoms and $R_1$ and $R_2$ have the same meanings as above except for $R_1$ representing a chain $-CH(R_8)-COOR_4$ in which $R_4$ represents a hydrogen atom or a chain $-CH_2-CO-NR_5R_6$, an amine of formula:

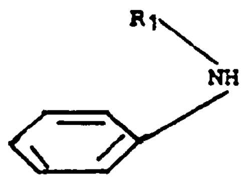

(V)

in which $R_1$ has the same meanings as above, is reacted with a derivative of formula:

$HOOC-R_2-NH-CO-R_3$ (XII)

in which $R_2$ and $R_3$ have the same meanings as above, and the product is isolated,
E. for the preparation of the compounds of formula (I) for which $R_3$ represents a phenylamino radical in which the phenyl ring is optionally substituted by an alkyl, alkoxy, nitro or alkylthio radical or by one or two halogen atoms and $R_1$ represents a chain $-CH(R_8)-COOR_4$ in which $R_4$ represents a hydrogen atom, a derivative of corresponding formula (I), for which $R_1$ represents a chain $-CH(R_8)-COOR_4$ in which $R_4$ represents an alkyl radical, is hydrolysed and then the product is isolated,
F. for the preparation of the compounds of formula (I) for which $R_3$ represents a phenylamino radical in which the phenyl ring is optionally substituted by an alkyl, alkoxy, nitro or alkylthio radical or by one or two halogen atoms and $R_1$ represents a chain $-CH_2-CO-NR_5R_8$, an acid of corresponding formula (I), for which $R_1$ represents a chain $-CH(R_8)-COOR_4$ in which $R_4$ represents a hydrogen atom, or a reactive derivative of this acid is reacted with an amine of formula:

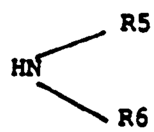

(VIII)

in which $R_5$ and $R_6$ have the same meanings as above, and then the product is isolated.
G. for the preparation of the compounds of formula (I) for which $R_3$ represents a phenylamino radical in which the phenyl ring is substituted by a hydroxyl radical, a compound of corresponding formula (I), for which $R_3$ represents a phenylamino radical in which the phenyl ring is substituted by an alkoxy radical, is dealkylated and then the product is isolated,
H. for the preparation of the compounds of formula (I) for which $R_1$ represents a chain $-CH(R_8)-COOR_4$ in which $R_4$ represents a phenyl radical, phenol is reacted with a compound of corresponding formula (I) for which $R_4$ represents a hydrogen atom and then the product is isolated.

## Patentansprüche

### Patentansprüche für folgende Vertragsstaaten : AT, BE, CH, DE, DK, FR, GB, IT, LI, LU, NL, SE

1. Verbindungen der Formel

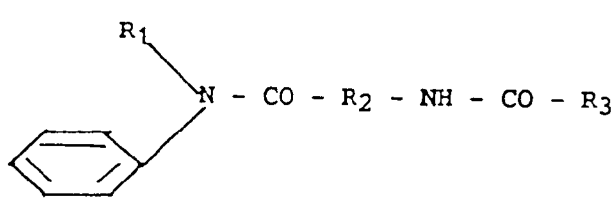

(I)

worin

- $R_1$ für
  - einen Phenylrest oder einen durch einen Alkyl-, Alkoxy-, Cyanorest oder ein Halogenatom substituierten Phenylrest,
  - eine -$CH(R_8)$-$COOR_4$-Kette, worin $R_4$ für ein Wasserstoffatom oder einen Alkyl-, Cycloalkyl-, Cycloalkylalkyl-, Phenylalkyl- oder Phenylrest steht, und $R_8$ für ein Wasserstoffatom oder einen Alkyl- oder Phenylrest steht,
  - eine -$CH_2$-CO-$NR_5R_8$-Kette, worin $R_8$ und $R_8$, die gleich oder verschieden sind, für einen Alkylrest stehen, oder mit den Stickstoffatom, an das sie gebunden sind, einen, gegebenenfalls durch einen Alkylrest substituierten, 1-Pyrrolidinylrest bilden, oder
  - einen Phenylalkylrest steht,
- $R_2$ für einen Methylen-, Ethylenrest oder den Rest -$CH(R_7)$-steht, worin $R_7$ für einen Alkyl-, Benzyl-, Alkylthioalkylrest, dessen Alkyleinheiten 1 oder 2 Kohlenstoffatome enthalten, oder einen Phenylrest steht,
- $R_3$ für einen 1- oder 2-Naphthyl- oder 2- oder 3-Indolyl- oder Phenylaminorest, dessen Phenylkern gegebenenfalls durch einen Alkyl-, Alkoxy-, Nitro-, Hydroxy- oder Alkylthiorest oder 1 oder 2 Halogenatome substituiert ist, steht,

mit der Maßgabe, daß, wenn $R_3$ für einen 1- oder 2-Naphthyl- oder 2- oder 3-Indolylrest steht, $R_7$ nicht für einen Benzyl-, Alkylthioalkyl- oder Phenylrest stehen kann, und daß, außer gegenteilig angegeben, die Alkyl- und Alkoxyreste und die Alkyl- und Alkoxyeinheiten 1 bis 4 Kohlenstoffatome in gerader oder verzweigter Kette enthalten, und wenn $R_2$ für den Rest -$CH(R_7)$- steht, ihre Razemate und Stereoisomere.

**2.** Verbindungen der Formel (I) nach Anspruch 1, **dadurch gekennzeichnet, daß**

- $R_1$ für
  - einen Phenylrest, der gegebenenfalls durch einen Alkoxyrest oder ein Halogenatom substituiert ist,
  - eine -$CH(R_8)$-$COOR_4$-Kette, worin $R_4$ für einen Alkyl- oder Cycloalkylalkylrest steht, und $R_8$ für ein Wasserstoffatom oder einen phenylrest steht, steht,
- $R_2$ und $R_3$ wie in Anspruch 1 definiert sind.

**3.** Verfahren zur Herstellung von Verbindungen der Formel (I) nach Anspruch 1, worin

- $R_2$ für einen Methylen-, Ethylenrest oder den Rest -$CH(R_7)$-, worin $R_7$ für einen Alkylrest steht, steht,
- $R_3$ für einen Phenylaminorest, dessen Phenylkern gegebenenfalls durch einen Alkyl-, Alkoxy-, Nitro-, Alkylthiorest oder 1 oder 2 Halogenatome substituiert ist, steht, und
- $R_1$ wie in Anspruch 1 definiert ist,

**dadurch gekennzeichnet, daß** man ein Derivat der Formel

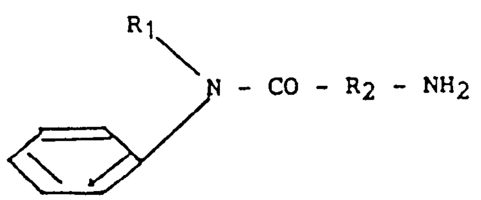

(II)

worin $R_2$ und $R_1$ wie vorstehend definiert sind, mit einem Phenylisocyanat, dessen Phenylkern gegebenenfalls durch einen Alkyl-, Alkoxy-, Nitro-, Alkylthiorest oder 1 oder 2 Halogenatome substituiert ist, umsetzt und dann das Produkt isoliert.

**4.** Verfahren zur Herstellung von Verbindungen der Formel (I) nach Anspruch 1, worin

- $R_2$ für einen Methylen-, Ethylenrest oder den Rest -$CH(R_7)$-, worin $R_7$ für einen Alkylrest steht, steht,
- $R_3$ für einen Phenylaminorest, dessen Phenylkern gegebenenfalls durch einen Alkyl-, Alkoxy-, Nitro-, Alkylthio- oder Hydroxylrest oder 1 oder 2 Halogenatome substituiert ist, steht, und
- $R_1$ wie in Anspruch 1 definiert ist,

**dadurch gekennzeichnet, daß** man eine Verbindung der Formel

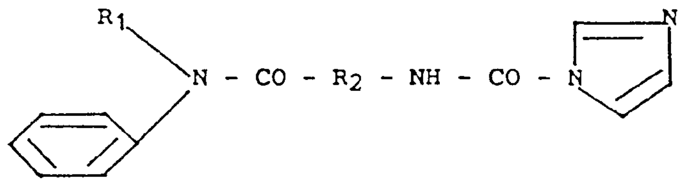 (X)

worin $R_1$ und $R_2$ wie vorstehend definiert sind, mit einem gegebenenfalls durch einen Alkyl-, Alkoxy-, Alkylthio-, Nitro-, Hydroxylrest oder 1 oder 2 Halogenatome substituierten Anilin, umsetzt und dann das Produkt isoliert.

5. Verfahren zur Herstellung von Verbindungen der Formel (I) nach Anspruch 1, worin
   - $R_2$ für einen Methylen-, Ethylenrest oder den Rest $-CH(R_7)-$, worin $R_7$ für einen Alkylrest steht, steht,
   - $R_3$ für einen 1- oder 2- Naphthyl- oder 2- oder 3-Indolylrest steht, und
   - $R_1$ wie in Anspruch 1 definiert ist,

   **dadurch gekennzeichnet, daß** man ein Derivat der Formel

   $HOOC-R_3$ (XI)

   worin $R_3$ für einen 1- oder 2-Naphthyl- oder 2- oder 3-Indolylrest steht- oder ein reaktives Derivat dieser Säure mit einem Derivat der Formel

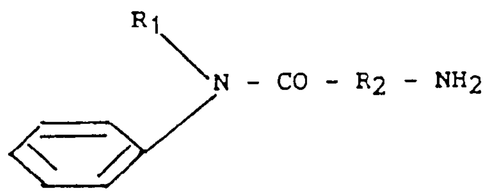 (II)

   worin $R_2$ und $R_1$ wie vorstehend definiert sind, umsetzt und dann das Produkt isoliert.

6. Verfahren zur Herstellung von Verbindungen der Formel (I) nach Anspruch 1, worin
   - $R_3$ für einen Phenylaminorest, dessen Phenylkern gegebenenfalls durch einen Alkyl-, Alkoxy-, Nitro-, Alkylthiorest oder 1 oder 2 Halogenatome substituiert ist, steht, und
   - $R_1$ und $R_2$ wie in Anspruch 1 definiert sind, mit Ausnahme der Bedeutung einer $-CH(R_8)-COOR_4$-Kette, worin $R_4$ für ein Wasserstoffatom steht, oder einer $-CH_2-CO-NR_5R_6$-Kette für $R_1$, **dadurch gekennzeichnet, daß** man ein Amin der Formel

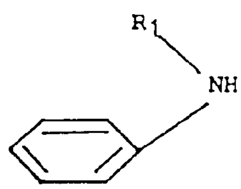 (V)

   worin $R_1$ wie vorstehend definiert ist, mit einem Derivat der Formel

   $HOOC-R_2-NH-CO-R_3$ (XII)

   worin $R_2$ und $R_3$ wie vorstehend definiert sind, umsetzt und dann das Produkt isoliert.

7. Verfahren zur Herstellung von Verbindungen der Formel (I) nach Anspruch 1, worin
   - $R_3$ für einen Phenylaminorest, dessen Phenylkern gegebenenfalls durch einen Alkyl-, Alkoxy-, Nitro-, Alkylthiorest oder 1 oder 2 Halogenatome substituiert ist, steht,
   - $R_2$ wie in Anspruch 1 definiert ist, und
   - $R_1$ für eine $-CH(R_8)-COOR_4$-Kette, worin $R_4$ für ein Wasserstoffatom steht, steht, **dadurch gekennzeichnet, daß** man ein entsprechendes Derivat der Formel (I), worin $R_1$ für eine $-CH(R_8)-COOR_4$-Kette, worin $R_4$ für einen Alkylrest steht, steht, hydrolysiert und dann das Produkt isoliert.

8. Verfahren zur Herstellung von Verbindungen der Formel (I) nach Anspruch 1, worin

- $R_3$ für einen Phenylaminorest, dessen Phenylkern gegebenenfalls durch einen Alkyl-, Alkoxy-, Nitro-, Alkylthiorest oder 1 oder 2 Halogenatome substituiert ist, steht,
- $R_2$ wie in Anspruch 1 definiert ist, und
- $R_1$ für eine $-CH_2-CO-NR_5R_5$-Kette steht,

**dadurch gekennzeichnet, daß** man eine entsprechende Säure der Formel (I), worin $R_1$ für eine $-CH(R_8)-COOR_4$-Kette, worin $R_4$ für ein Wasserstoffatom steht, steht, oder ein reaktives Derivat dieser Säure mit einem Amin der Formel

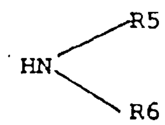

(VIII)

worin $R_5$ und $R_5$ wie in Anspuch 1 definiert sind, umsetzt und dann das Produkt isoliert.

9. Verfahren zur Herstellung von Verbindungen der Formel (I) nach Anspruch 1, worin
   - $R_3$ für einen Phenylaminorest, dessen Phenylkern durch eine Hydroxylgruppe substituiert ist, steht, und
   - $R_1$ und $R_2$ wie in Anspruch 1 definiert sind,

   **dadurch gekennzeichnet, daß** man eine entsprechende Verbindung der Formel (I), worin $R_3$ für einen Phenylaminorest, dessen Phenylkern durch einen Alkoxyrest substituiert ist, steht, desalkyliert und dann das Produkt isoliert.

10. Verfahren zur Herstellung von Verbindungen der Formel (I) nach Anspruch 1, worin $R_1$ für eine $-CH(R_8)-COOR_4$-Kette, worin $R_4$ für einen Phenylrest steht, steht, **dadurch gekennzeichnet, daß** man das Phenol mit einer entsprechenden Verbindung der Formel (I), worin $R_4$ für ein Wasserstoffatom steht, umsetzt und dann das Produkt isoliert.

11. Medikamente, **dadurch gekennzeichnet, daß** sie als Wirkstoff mindestens eine Verbindung der Formel (I) nach Anspruch 1, in Reinform oder zusammen mit einem oder mehreren verträglichen und pharmazeutisch annehmbaren Verdünnungsmitteln oder Adju-vantien enthalten.

12. Medikamente nach Anspruch 11zur Behandlung oder Prävention von Störungen im Zusammenhang mit CCK im Nervensystem oder Magen-Darm-Trakt.

**Patentansprüche für folgende Vertragsstaaten : ES, GR**

1. Verfahren zur Herstellung von Verbindungen der Formel

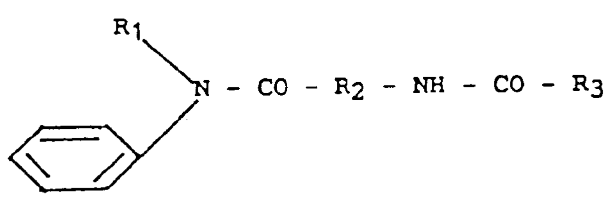

(I)

worin
- $R_1$ für
  - einen Phenylrest oder einen durch einen Alkyl-, Alkoxy-, Cyanorest oder ein Halogenatom substituierten Phenylrest,
  - eine $-CH(R_8)-COOR_4$-Kette, worin $R_4$ für ein Wasserstoffatom oder einen Alkyl-, Cycloalkyl-, Cycloalkylalkyl-, Phenylalkyl- oder Phenylrest steht, und $R_5$ für ein Wasserstoffatom oder einen Alkyl- oder Phenylrest steht,
  - eine $-CH_2-CO-NR_5R_5$-Kette, worin $R_5$ und $R_5$, die gleich oder verschieden sind, für einen Alkylrest stehen, oder mit den Stickstoffatom, an das sie gebunden sind, einen, gegebenenfalls durch einen Alkylrest substituierten, 1-Pyrrolidinylrest bilden, oder

- einen Phenylalkylrest steht,
- $R_2$ für einen Methylen-, Ethylenrest oder den Rest -CH($R_7$)-steht, worin $R_7$ für einen Alkyl-, Benzyl-, Alkylthioalkylrest, dessen Alkyleinheiten 1 oder 2 Kohlenstoffatome enthalten, oder einen Phenylrest steht,
- $R_3$ für einen 1- oder 2-Naphthyl- oder 2- oder 3-Indolyl- oder Phenylaminorest, dessen Phenylkern gegebenenfalls durch einen Alkyl-, Alkoxy-, Nitro-, Hydroxy- oder Alkylthiorest oder 1 oder 2 Halogenatome substituiert ist, steht,

mit der Maßgabe, daß wenn $R_3$ für einen 1- oder 2-Naphthyl- oder 2- oder 3-Indolylrest steht, $R_7$ nicht für einen Benzyl-, Alkylthioalkyl- oder Phenylrest stehen kann, und daß, außer gegenteilig angegeben, die Alkyl- und Alkoxyreste und die Alkyl- und Alkoxyeinheiten 1 bis 4 Kohlenstoffatome in gerader oder verzweigter Kette enthalten, und wenn $R_2$ für den Rest -CH($R_7$)- steht, ihrer Razemate und Stereoisomere, **dadurch gekennzeichnet, daß** man

A) zur Herstellung von Verbindungen der Formel (I), worin $R_2$ für einen Methylen-, Ethylenrest oder den Rest -CH($R_7$)-, worin $R_7$ für einen Alkylrest steht, steht, $R_3$ für einen Phenylaminorest, dessen Phenylkern gegebenenfalls durch einen Alkyl-, Alkoxy-, Nitro-, Alkylthiorest oder 1 oder 2 Halogenatome substituiert ist, steht, ein Derivat der Formel

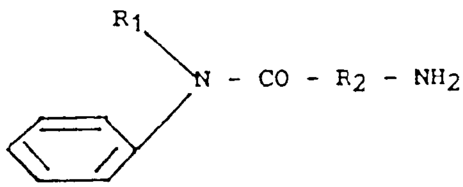

(II)

worin $R_1$ und $R_2$ wie vorstehend definiert sind, mit einem Phenylisocyanat, dessen Phenylkern gegebenenfalls durch einen Alkyl-, Alkoxy-, Nitro-, Alkylthhiorest oder 1 oder 2 Halogenatome substituiert ist, umsetzt und dann das Produkt isoliert,

B) zur Herstellung von Verbindungen der Formel (I), worin
$R_2$ für einen Methylen-, Ethylenrest oder den Rest -CH($R_7$)-, worin $R_7$ für einen Alkylrest steht, steht, $R_3$ für einen Phenylaminorest, dessen Phenylkern gegebenenfalls durch einen Alkyl-, Alkoxy-, Nitro-, Alkylthio-, Hydroxylrest oder 1 oder 2 Halogenatome substituiert ist, steht, eine Verbindung der Formel

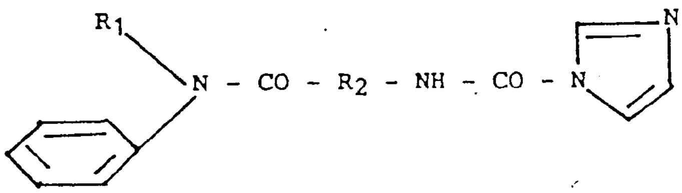

(X)

worin $R_1$ und $R_2$ wie vorstehend definiert sind, mit einem gegebenenfalls durch einen Alkyl-, Alkoxy-, Alkylthio-, Nitro-, Hydroxylrest oder 1 oder 2 Halogenatome substituierten Anilin umsetzt und dann das Produkt isoliert,

C) zur Herstellung von Verbindungen der Formel (I), worin $R_2$ für einen Methylen-, Ethylenrest oder den Rest -CH($R_7$)-, worin $R_7$ für einen Alkylrest steht, steht, und $R_3$ für einen 1- oder 2- Naphthyl- oder 2- oder 3-Indolylrest steht, ein Derivat der Formel

$$HOOC\text{-}R_3 \qquad (XI)$$

worin $R_3$ für einen 1- oder 2-Naphthyl- oder 2- oder 3-Indolyl- rest steht, oder ein reaktives Derivat dieser Säure mit einem Derivat der Formel

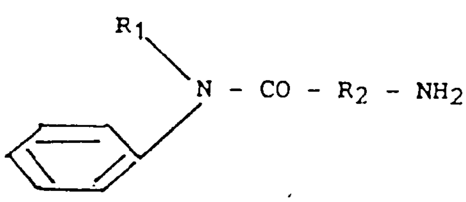

(II)

worin $R_1$ und $R_2$ wie vorstehend definiert sind, umsetzt und dann das Produkt isoliert,

D) zur Herstellung von Verbindungen der Formel (I), worin $R_3$ für einen Phenylaminorest, dessen Phenylkern gegebenenfalls durch einen Alkyl- , Alkoxy-, Nitro-, Alkylthiorest oder 1 oder 2 Halogenatome substituiert ist, steht, und $R_1$ und $R_2$ wie vorstehend definiert sind, mit Ausnahme der Bedeutung einer $-CH(R_8)-COOR_4$-Kette, worin $R_4$ für ein Wasserstoffatom steht, oder einer $-CH_2-CO-NR_5R_8$-Kette für $R_1$, ein Amin der Formel

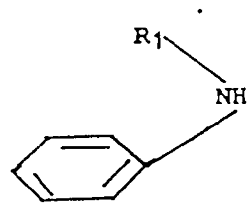

(V)

worin $R_1$ wie vorstehend definiert ist, mit einem Derivat der Formel

$$HOOC-R_2-NH-CO-R_3 \qquad (XII)$$

worin $R_2$ und $R_3$ wie vorstehend definiert sind, umsetzt und dann das Produkt isoliert,

E) zur Herstellung von Verbindungen der Formel (I), worin $R_3$ für einen Phenylaminorest, dessen Phenylkern gegebenenfalls durch einen Alkyl-, Alkoxy-, Nitro-, Alkylthiorest oder 1 oder 2 Halogenatome substituiert ist, steht, und $R_1$ für eine $-CH(R_8)-COOR_4$-Kette, worin $R_4$ für ein Wasserstoffatom steht, steht, ein entsprechendes Derivat der Formel (I), worin $R_1$ für eine $-CH(R_8)-COOR_4$-Kette, worin $R_4$ für einen Alkylrest steht, steht, hydrolysiert und dann das Produkt isoliert,

F) zur Herstellung von Verbindungen der Formel (I), worin $R_3$ für einen Phenylaminorest, dessen Phenylkern gegebenenfalls durch einen Alkyl-, Alkoxy-, Nitro-, Alkylthiorest oder 1 oder 2 Halogenatome substituiert ist, steht, und $R_1$ für eine $-CH_2-CO-NR_5R_8$-Kette steht, eine entsprechende Säure der Formel (I), worin $R_1$ für eine $-CH(R_8)-COOR_4$-Kette, worin $R_4$ für ein Wasserstoffatom steht, steht, oder ein reaktives Derivat dieser Säure, mit einem Amin der Formel

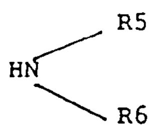

(VIII)

worin $R_5$ und $R_6$ wie vorstehend definiert sind, umsetzt und dann das Produkt isoliert,

G) zur Herstellung von Verbindungen der Formel (I), worin $R_3$ für einen Phenylaminorest, dessen Phenylkern durch eine Hydroxylgruppe substituiert ist, steht, eine entsprechende Verbindung der Formel (I), worin $R_3$ für einen Phenylaminorest, dessen Phenylkern durch einen Alkoxyrest substituiert ist, steht, desalkyliert und dann das Produkt isoliert,

H) zur Herstellung von Verbindungen der Formel (I), worin $R_1$ für eine $-CH(R_8)-COOR_4$-Kette, worin $R_4$ für einen Phenylrest steht, steht, das Phenol mit einer entsprechenden Verbindung der Formel (I), worin $R_4$ für ein Wasserstoffatom steht, umsetzt und dann das Produkt isoliert.